Europäisches Patentamt

European Patent Office

Office européen des brevets

(19)

(11) **EP 0 920 908 A1**

(12) **EUROPÄISCHE PATENTANMELDUNG**

(43) Veröffentlichungstag:
**09.06.1999 Patentblatt 1999/23**

(51) Int. Cl.⁶: **B01J 23/83**, B01J 35/02,
C07C 17/156, B01J 21/06

(21) Anmeldenummer: 98121259.0

(22) Anmeldetag: 07.11.1998

(84) Benannte Vertragsstaaten:
**AT BE CH CY DE DK ES FI FR GB GR IE IT LI LU
MC NL PT SE**
Benannte Erstreckungsstaaten:
**AL LT LV MK RO SI**

(30) Priorität: 24.11.1997 DE 19751962

(71) Anmelder:
**DEGUSSA AKTIENGESELLSCHAFT
60311 Frankfurt (DE)**

(72) Erfinder:
• **Müller, Herbert Dr.
67122 Altrip (DE)**
• **Bösing, Stefan
55246 Mainz-Kostheim (DE)**
• **Schmidhammer, Ludwig Dr.
84533 Haiming (DE)**
• **Frank, Albin Dr.
84489 Burghausen (DE)**
• **Haselwarter, Klaus
84547 Emmerting (DE)**

(54) **Trägerkatalysator, Verfahren zu dessen Herstellung sowie Verwendung bei der Oxychlorierung von Ethylen**

(57) Ein Trägerkatalysator enthält Kupfer(II)verbindungen (a), Alkalimetallverbindungen (b), Oxide des Lanthans, Cers, Praseodyms, Neodyms und/oder Samariums (c1), und Zirkondioxid (c2). Das Trägermaterial ist $\gamma$- und/oder $\Theta$aluminiumoxid (d) mit einem Gesamtporenvolumen im Bereich von 0,65 bis 1,2cm³/g. Der Trägerkatalysator liegt vor als zylindrischer Hohlkörper (f) mit wenigstens einem Durchtrittskanal, wobei für einen Durchmesser $d_1$ von bis zu 6mm das Verhältnis Höhe h zu Außendurchmesser $d_1$ kleiner als 1,5 ist und für einen Durchmesser über 6mm das Verhältnis $h/d_1$ kleiner als 0,6 ist. Die Trägerkatalysatoren werden hergestellt durch:

I   Beaufschlagung der schon geformten Trägermaterialien (d)(Geometrie (f)) mit löslichen Vorläufern der Komponenten (c1) und (c2); Überführung der Vorläufer-Verbindungen in die oxidische Form; Beladung der oxidisch beaufschlagten Trägermaterialien mit den Komponenten (a) und (b).

II  Formung einer Mischung aus den Komponenten (c1,c2) und (d) zur Geometrie (f); Beladung der geformten Trägermaterialien mit den Komponenten (a) und (b).

Der Katalysator ist zur Herstellung von 1,2-Dichlorethan durch Oxichlorierung von Ethylen geeignet.

EP 0 920 908 A1

**Beschreibung**

Die Erfindung betrifft einen geformten Trägerkatalysator zur Oxichlorierung von Ethylen, der bei relativ niedrigen Reaktionstemperaturen und großen Raumströmungsgeschwindigkeiten einen hohen Chlorwasserstoffumsatz bei gesteigerter Reaktionsselektivität erzielt und seine geometrische Formgebung und Aktivität über einen langen Zeitraum bewahrt. Der erfindungsgemäße Trägerkatalysator enthält Kupfer- und Alkaliionen auf speziell geformten Aluminiumoxid-Trägern mit erhöhten Porenvolumina speziell im Mesoporenbereich, die durch oxidische Zusätze gegen thermisch bedingten Zerfall formstabilisiert sind. Die Erfindung richtet sich auch auf ein Verfahren zur Herstellung solcher geträgerter Katalysatoren sowie die Verwendung dieser Trägerkatalysatoren insbesondere bei der selektiven Oxichlorierung von Ethylen mit Luft, mit Sauerstoff angereicherter Luft oder mit reinem Sauerstoff unter ethylenreicher Kreisgasführung.

Die Oxichlorierung von Ethylen ist ein bekanntes großtechnisch ausgeübtes Verfahren, das in Fließbett- und Festbett-Reaktoren durchgeführt wird. Trotz gleichmäßigerer Temperaturverteilung in der Reaktionszone sind die Nachteile des Fließbettverfahrens, wie Rückvermischungs- und Abriebprobleme sowie gewisse Schwierigkeiten im Fließverhalten des Katalysators, die zu einem Zusammenkleben der Katalysatorpartikel führen können, worunter vor allem die Reaktionsselektivität leidet, nicht zu übersehen. So kann beispielsweise der technologische Vorteil einer Sauerstoff-Kreisgasfahrweise mit bis zu 80 Vol % Ethylen im Kreisgas, wodurch die freiwerdende Reaktionsenthalpie entsprechend dem aktuellen Stand der bestverfügbaren Technologie optimal abgeführt wird, in einer Fließbett-Oxichlorierung nicht zur Anwendung kommen.

Beim Festbettverfahren, bei dem darüberhinaus auch die Reationspartner exakter kontrolliert werden können, ist hingegen die Sauerstoff-Kreisgasfahrweise mit hohem Ethylengehalt im Kreisgas problemlos durchführbar. Die Unterdrückung der Nachteile des Festbettverfahrens, wie insbesondere das Auftreten lokaler Temperaturnester (sog. Hot spots), erhöhter Druckabfall über die Reaktoren sowie allmählicher Zerfall des geformten Trägerkatalysators durch Kokseinlagerungen und thermisch bedingte Langzeiteffekte, die die Aktivität und Selektivität sowie die Katalysatorlebensdauer negativ beeinflussen, ist Gegenstand zahlreicher literaturbekannter Untersuchungen und Verbesserungsvorschläge, wie die Zugabe aktivitäts- und selektivitätssteigender Promotoren, der Zusatz von Thermostabilisatoren zum Trägermaterial, die Auswahl strömungsgünstiger bzw. thermodynamisch und reaktionskinetisch vorteilhafter Katalysator-Geometrien bzw.-Porositäten und verfahrenstechnische Verbesserungen durch Übergang auf die sogenannten Multireaktortechnologie mit Aufsplitten des Chorwasserstoffes und/oder der Luft bzw. des Sauerstoffs auf die einzelnen in Serie geschalteten Reaktoren mit und ohne Kreisgasführung.

Zum speziellen Stand der Technik wurden folgende Druckschriften genannt:

- Allen, J.A., Clark, A.J., Rev. Pure and Appl. Chem. 21, 148 (1971) = D1;
- DE-A-17 68 453 = D2;
- Allen, J.A., Clark A.J., J. Appl. Chem., 26, 327 (1966) = D3;
- DE-A-20 50 061 = D4;
- US- 4,446,249 = D5;
- FR-2021986 = D6;
- Dotson, R.L., J. Catalysis 33, 210 (1974) = D7;
- Villadsen, J., Livbjerg, H., Catal. Rev. Sci. Eng. 17 (2), 203 (1978)= D8;
- Shatchortswa, G.A. et al., Kinet, i Katal. 11, 1224 (1970) = D9;
- Ruthren, D.M., Kennedy, C.N., J. Inorg. Nucl. Chem. 30, 931 (1965) = D10;
- Dirksen, F., Chemie-Technik 12 (1983) Nr. 6, 36 - 43 = D11;
- EP-A-0 582 165 = D12;
- EP-A- 0 775 522 = D 13; und
- WO 96/40431 = D 14

Aus der D1 sind eine Vielzahl von Promotoren bzw. Aktivatoren, wie die Oxide bzw. Chloride von Lanthan, Platin, Zirkonium, Uran, Cer, Thorium, Titan, Tantal, Rhodium, Molybdän, Ruthenium, Wolfram, Europium sowie Didymium-Gemische Seltener Erden bekannt, die teilweise sowohl die reine Deacon-Reaktion als auch die Oxichlorierungsreaktion günstig beeinflussen sollen.

Die D2 beschreibt einen Fließbett-Oxichlorierungskatalysator auf der Basis von Kieselsäure als Träger, der zusätzlich zu Kupferchlorid und Alkalichlorid neben Chloriden der Seltenen Erden oder Chloriden von Scandium, Zirkon, Thorium und Uran auch Yttriumchlorid enthält. Yttrium-III-chlorid reagiert zwar mit Sauerstoff unter Chlorentwicklung zu $Y_2O_3$, dabei entsteht jedoch ein gasundurchlässiger Oxidfilm (D3). Somit ist das System $Yl_3/Y_2O_3$ für die Festbett-Katalyse wenig geeignet, weil durch den filmartigen Oxidüberzug die Katalysatoroberfläche verklebt und die Geschwindigkeitsrate der Reaktion zwischen $Yl_3$ und Sauerstoff sehr schnell gebremst wird. Beim Fließbettverfahren hingegen erfolgt eine mechanische Zerstörung des filmartigen Oxidüberzugs durch ständig aneinander-reibende Katalysatorpartikel.

Die D4 beansprucht einen Trägerkatalysator für die Fließbett- oder Festbett-Oxichlorierung, der neben Kupferchlorid Verbindungen der Seltenen Erdmetalle mit Ordnungszahl 62 und darüber und/oder Verbindungen von Yttrium auf aktiver Tonerde als Trägermaterial enthält. Widersprüchlich zu D2 ist dabei, daß die Anwesenheit von Alkalimetallen schädlich ist, weil sie die Aktivität der Katalysatoren verringern.

Gemäß D5 und D6 verwendet man Zusätze von Verbindungen der Seltenen Erdmetalle insbesondere Verbindungen des Lanthans bei Fließbettverfahren, um ein Agglomerieren der Fließbett-Katalysatorpartikel zu verhindern.

Der gleiche Effekt der Lanthanidenmetallverbindungen wird von R.L. Dotson in D7 beschrieben.

Nach D8 erhöht jedoch Lanthan-III-chlorid die Sublimationsrate von Kupferchlorid/Kaliumchlorid-Systemen, wodurch solche Katalysatoren mit der Zeit an Aktivität verlieren.

In der D9 wird vermutet, daß Lanthan aufgrund seiner starken Komplexbildungstendenz Chloridionen vom System Kupferchlorid/Kaliumchlorid abzieht, wodurch der Kalium-Kupferchlorokomplex chloridärmer wird und somit sich die Kupferchlorid-Flüchigkeit erhöht.

Gemäß D10 beruht die aktivierende Wirkung von Lanthan-III-chlorid nur auf einer Erhöhung des pre-exponentiellen Frequenzfaktors der Arrhenius-Gleichung, ohne die Aktivierungsenergie herabzusetzen, indem es die geschwindigkeitsbestimmende Reoxidation der intermediär sich ausbildenden Kupfer-I-species beschleunigt. Da dieser Frequenzfaktor jedoch erheblich weniger als die Reaktionsgeschwindigkeitskonstante k von der Temperatur abhängig ist, ist der katalytische Effekt von Lanthan-III-chlorid insgesamt schwächer ausgeprägt.

Zur Verbesserung der Temperaturstabilität bzw. Wärmefestigkeit eines Oxichlorierungskatalysators auf Basis gammma-Aluminiumoxid als Träger, das heißt zur Erhöhung seiner Widerstandsfähigkeit gegenüber erhöhten Temperaturen während seiner Einsatzdauer ohne Veränderung der physikalischen Struktur der transition aluminia Matrix, verwendet man unter anderem Zusätze von Lanthanoxid oder Thoriumoxid (D11) und andere Oxide der Seltenen Erdmetalle (D4), wobei allerdings allein schon das zur Gewinnung des aktivierten Aluminumoxides verwandte Aluminiumoxidhydrat für die Temperaturstabilität des gewonnenen porösen Festkörpers von entscheidender Bedeutung ist. Das durch Böhmit-Dehydratisierung gewonnene gamma-Aluminiumoxid ist in Bezug auf eine Wärmebehandlung am stabilsten, wobei bei der Einwirkung erhöhter Temperaturen unter hydrothermalen Bedingungen einer Oxichlorierungsreaktion während seiner Einsatzdauer eine allmähliche Umwandlung zu dem katalytisch inaktiven aber thermodynamisch stabilem alpha-Aluminiumoxid erfolgt, vermutlich nach Teilrehydratisierung zu Böhmit und Diaspor (Gleichgewicht) der schon bei 420°C in alpha-Aluminiumoxid übergeht, während durch thermischen Abbau von gamma-Aluminiumoxid erst bei 1000°C alpha-Aluminiumoxid entsteht. Dabei wandelt sich das tetragonal kristallisierte gamma-Aluminiumoxid in Spinellform in das hexagonal rhomboedrische alpha-Aluminiumoxid um. Diese Veränderung der physikalischen Struktur bzw. Morphologie bewirkt einen allmählichen Zerfall der Katalysatorformlinge, wodurch sich der Druckabfall über der Reaktorschüttung bis hin zur Bettverdichtung erhöhen kann und aufgrund unterschiedlicher Zerfallsraten die Gasverteilung über den Reaktorquerschnitt immer ungleichmäßiger wird. Beides hat zur Folge, daß der zerfallene Katalysator vorzeitig ausgewechselt werden muß.

Daneben ist bekannt, daß die mechanische Festigkeit von Trägerkatalysatoren von deren Geometrie und Porosität abhängt, wobei man ganz allgemein annimmt, daß dickwandige Trägergeometrien mit großen Durchmessern bzw. Träger mit relativ geringem Porenvolumen mechanisch am stabilsten sind.

Aus der D12 sind ein Katalysator und ein Verfahren zur Oxichlorierung von Ethylen in Dichlorethan bekannt; der Katalysator umfaßt einen Träger, der eine aktive Metall-Zusammensetzung aufweist, die 2 bis 8 Gew.-% Kupfer als Chlorid - oder in Form anderer Kupfersalze -, 0,2 bis 2 Gew.-% eines Alkalimetalls (Alkalimetalle), 0,1 bis 9 Gew.-% eines Seltenerdmetalls (Seltenerdmetalle) und 0,05 bis 4 Gew.-% eines Metalls (Metalle) der Gruppe IIA des Periodensystems der Elemente (IUPAC 1970) umfaßt, wobei alle Gew.-% auf das Gesamtgewicht der Katalysator-Zusammensetzung bezogen sind, worin alle Metalle auf dem Träger abgeschieden werden, und die Katalysator-Zusammensetzung eine spezifische Oberfläche im Bereich von 20 bis 220 $m^2$/g aufmisst. Per Träger ist Aluminiumoxid mit einer Schüttdichte im Bereich von 0,8 bis 1,1 g/cm$^3$ und einem Porenvolumen zwischen 0,2 und 0,5 ml/g.

Die D13 betrachtet Katalysatoren für die Oxichlorierung von Ethylen in Form von hohlzylindrischen Granulaten mit wenigstens drei Durchgangskanälen. Es wird ein Herstellverfahren offenbart, bei dem Katalysatoren resultieren, die eine größere Porosität und eine engere Porenradiusverteilung aufweisen. Die Porosität liegt im Bereich von 0,2 bis 0,5 cm$^3$/g, während die BET-Oberfläche im Bereich von 80 bis 380 $m^2$/g ist. Ähnlich wie in D14, welche wagenradförmige Katalysatoren für die Oxichlorierung offenbart.

Es hat sich jedoch gezeigt, daß die bisher gegebenen Hinweise und Annahmen, die teilweise widersprüchlich sind, in deren Mehrzahl nicht ausreichen, um zwingenderweise allen Belangen gerecht werdende Trägerkatalysatoren zu erzielen.

Angesichts der hierin angegebenen und diskutierten Standes der Technik bestand daher die Aufgabe einen Trägerkatalysator für die Oxichlorierung von Ethylen zu schaffen, der über eine verbesserte Aktivität verfügt.

Eine weitere Aufgabe der Erfindung bestand darin, einen Trägerkatalysator für die Oxichlorierung von Ethylen zur Verfügung zu stellen, der über eine verbesserte Selektivität verfügt.

Noch eine weitere Aufgabe bestand in der Bereitstellung eines Trägerkatalysators mit verbesserter Temperaturstabilität insbesondere gegen örtliche Überhitzung für die Oxichlorierung von Ethylen.

Weiters bestand die Aufgabe, die Aktivität, Selektivität und Temperaturstabilität insbesondere gegen örtliche Überhitzung der bisher bekannten Trägerkatalysatoren für die Oxichlorierung von Ethylen zu verbessern, um im Vergleich zu den Trägerkatalysatoren des Standes der Technik mit strömungsgünstigen und thermodynamisch wie reaktionskinetisch vorteilhaften Geometrien und Porositäten eine verbesserte Eigenschaftskombination der erfindungsgemäßen Trägerkatalysatoren hinsichtlich Aktivität, Selektivität und Formstabilität über einen langen Zeitraum zu erhalten.

Darüber hinaus war es Aufgabe der Erfindung ein einfaches und leicht durchführbares Verfahren zur Herstellung eines Trägerkatalysators mit verbesserter Aktivität, Selektivität, Temperaturstabilität und/oder verbesserter Langzeitstabilität der Aktivität, Selektivität und/oder Formstabilität anzugeben. Schließlich soll auch die Verwendung von verbesserten Trägerkatalysatoren aufgezeigt werden.

Gelöst werden diese Aufgaben sowie weitere nicht näher genannte, jedoch aus den einleitenden Erörterungen ohne weiteres sich erschließende oder wie selbstverständlich sich ergebende Aufgaben durch einen Trägerkatalysator mit den Merkmalen des Anspruches 1.

Zweckmäßige Ausführungsformen des erfindungsgemäßen Trägerkatalysators wurden in den auf den Anspruch 1 rückbezogenen Produktansprüchen unter Schutz gestellt. Hinsichtlich des Herstellverfahrens lösen die Gegenstände der Ansprüche 16 und 17 die der Erfindung zugrunde liegenden Probleme.

Gegenstand des Anspruches 18 ist die Verwendung der neuen Trägerkatalysatoren gemäß der Erfindung, während Anspruch 19 ein verwendungsgemäßes Verfahren schützt.

Dadurch, daß ein Trägerkatalysator

a) 0,5 - 15 Gew.-% einer oder mehrerer $Cu^{II}$-Verbindungen, wobei sich die Mengenangabe auf Kupfermetall bezieht,

b) 0,1 - 8 Gew.-% einer oder mehrerer Alkalimetallverbindungen, wobei sich die Mengenangabe auf Alkalimetall bezieht.

c) 0,1 - 10 Gew.-% eines Oxidgemisches, welches aus

c1) 80 - 95 Mol % von Oxiden der Ceriterden mit den Ordnungszahlen 57 bis 62, ausgenommen Promethium, und

c2) 5 - 20 Mol % Zirkondioxid, besteht, wobei c1) und c2) zusammen 100 Mol % ergeben müssen und wobei sich die Mengenangabe von c) auf die Oxide des Gemisches bezieht, und

d) den Rest auf 100 Gew.-% als Trägermaterial $\gamma$- und/oder $\vartheta$-Aluminiumoxid; aufweist, wobei

e) das Trägermaterial d) ein Gesamtporenvolumen im Bereich von 0,65 bis 1,2 $cm^3$/g aufweist; und wobei

f) der Trägerkatalysator als zylindrischer Hohlkörper mit wenigstens einem Durchtrittskanal vorliegt, wobei für Durchmesser $d_a$ von bis zu 6 mm das Verhältnis von Höhe h zu Außendurchmesser $d_a$ kleiner 1,5 ist und für Durchmesser $d_a$ über 6 mm das Verhältnis h/$d_a$ kleiner 0,6 ist, gelingt es in nicht ohne weiteres vorab überschaubarer Weise eine ganze Reihe von Vorteilen miteinander zu kombinieren. Die erfindungsgemäßen Trägerkatalysatoren zeigen oder ermöglichen:

- niedrige Druckverluste;
- niedere Schüttdichten;
- relativ große äußere Oberflächen pro Einheitsvolumen eines Reaktionsgefäßes;
- gleichmäßige Durchströmung des oder der Durchtrittskanals bzw. - kanäle;
- Erzeugung hoher Reaktionsgasturbulenzen im Inneren der Katalysatorformlinge;
- Erzeugung hoher Reaktionsgasturbulenzen um den Katalysatorformkörper herum;
- Erhöhung der axialen und radialen Vermischung, das heißt Stofftransport entgegen der Strömungsrichtung, unter Einfluß eines Konzentrationsgradienten;
- Steigerung und Erleichterung des Diffusionsvermögens der gasförmigen Reaktanden in den Kanälen;
- Steigerung und Erleichterung der gasförmigen Reaktanden in den Katalysatorporen infolge Mesoporendominanz;
- hohe Turbulenzen der Reaktionsgase an den Katalysatorformlingen und im Reaktionsraum verbessern die nachteiligen Wärmeabführungsprobleme, die aufgrund reduzierter Wärmeaustauschkoeffizienten zwischen Katalysatorformlingen und Reaktionsgas auftreten;
- Erzielung höherer Raum-Zeit-Ausbeuten;
- Erhöhung des Umsatzes;
- Erhöhung der Selektivität;
- Erhöhung der Katalysatorstandzeit;

Insbesondere sind hierbei folgende Effekte als für den Fachmann überraschend zu bezeichnen:

Es ist bekannt, daß ein hoher Lückengrad die katalytisch aktive Masse pro Einheit Reaktionsvolumen verringert. Es war daher nicht zu erwarten, daß gerade die erfindungsgemäßen Katalysatorgeometrien trotz ihrer relativ großen geometrischen Oberfläche pro Reaktorschüttvolumen, aber andererseits sehr hohem geometriebedingten Lückengrads, mit dazu beitragen, daß zusammen mit den übrigen Merkmalen der Erfindung eine Aktivitätssteigerung bei der Oxichlorierung erzielt wird.

Der temperaturstabilisierende Effekt von oxidischen Zusätzen von Ceriterdenmetallen allein unter Oxichlorierungsbedingungen hat nur eine geringe Wirkung. Ein Zusatz von Zirkondioxid allein ist völlig wirkungslos. Daher konnte auch nicht erwartet werden, daß mit dem erfindungsgemäßen Trägerkatalysator dennoch eine Verbesserung der Aktivität und Selektivität sowie der Form- und Wärmefestigkeit unter Langzeitwirkung erreicht wird.

Die Steigerung der Aktivität und Selektivität bei gleichzeitiger Erhöhung der Form- und Wärmefestigkeit unter Oxichlorierungsbedingungen zeigt sich nur, wenn Trägerkatalysatoren alle Merkmale des Anspruchs 1 aufweisen. Alle beanspruchten Parameter müssen gleichzeitig zugegen sein, um die bereits erwähnten Nachteile der Einzelparameter durch die erfindungsgemäße Merkmalskombination deutlich überzukompensieren, so daß letzlich eine für den Fachmann nicht zu erwartende Steigerung der Aktivität und Selektivität sowie der Form- und Wärmefestigkeit resultiert, die zudem über einen langen Zeitraum aufrecht erhalten bleibt. So verschlechtert sich beispielsweise die Temperaturstabilität der beanspruchten Katalysatorformlinge schon beträchtlich, wenn der Trägerkatalysator abweichend von der erfindungsgemäßen Merkmalskombination ein Gesamtporenvolumen von nur 0,5 $cm^3$/g aufweist.

Der erfindungsgemäße Trägerkatalysator basiert auf $\gamma$- und/oder $\vartheta$-Aluminiumoxid als Trägermaterial. Diese Aluminiumoxid-Modifikationen sind dem Fachmann geläufig, kommerziell erhältlich und damit verfügbar. Das Trägermaterial d) kann $\gamma$-$Al_2O_3$ sein, $\vartheta$-$Al_2O_3$ oder eine Mischung dieser beiden Modifikationen des $Al_2O_3$. In zweckmäßiger Abwandlung kennzeichnet sich der Trägerkatalysator der Erfindung dadurch, daß das Trägermaterial d) $\gamma$-$Al_2O_3$ ist. Der Anteil des Trägermaterials d) am Trägerkatalysator der Erfindung ist so bemessen, daß die Komponenten a)-d) zusammen 100 Gewichtsprozent ergeben. Üblicherweise liegt der Anteil des Trägermaterials d) rechnerisch bezogen auf Aluminiumoxid, im Bereich zwischen 76 und 99,3 Gewichtsprozent. Günstig sind Mengen im Bereich von 78 - 95 Gew.-%. Besonders zweckmäßig liegt der Anteil zwischen 79 und 90 Gew.%. Insbesondere bevorzugt ist der Bereich zwischen 85 und 94,5 Gew.%. Noch zweckmäßiger sind 87,5 - 92,5 Gew.-%.

Der erfindungsgemäße Trägerkatalysator enthält als Komponente c) ein Oxidgemisch. Dieses Oxidgemisch dient zur Modifizierung und Verbesserung der Eigenschaften des Trägermaterials d). Das Oxidgemisch c) ist im erfindungsgemäßen Trägerkatalysator in einer Menge im Bereich von 0,1 bis 10 Gewichtsprozent, bezogen auf das Gesamtgewicht der Komponenten a) bis d), enthalten. Bevorzugt ist der Anteil der Komponente c) im Bereich von 0,5 bis 10 Gew.-%, zweckmäßig sind 2 bis 8 Gew.-%, insbesondere vorteilhaft 3 bis 7, ganz besonders bevorzugt 5 bis 6 Gew.-% der Komponente c) im Trägerkatalysator a) bis d) (jeweils wt/wt) enthalten.

Bei der Komponente c) handelt es sich um eine Mischung aus den Komponenten c1) und c2). Die Komponente c2) ist Zirkondioxid. Die Komponente c1) ist ein oder mehrere Ceriterdenoxide von Elementen mit der Ordnungszahl 57 bis 62 (ausgenommen Promethium mit der Ordungszahl 61). Zu Oxiden, die als Komponente c2) fungieren gehören somit Oxide des Lanthan, Cer, Praseodym, Neodym und/oder Samarium.

Hiervon sind als Komponente c2) Oxide des Lanthan, Cer und/oder Samarium bevorzugt. Besonderes bevorzugt sind Oxide des Cer. Ganz besonders zweckmäßig ist die Komponente c2) Cerdioxid.

Das Verhältnis der Komponenten c1) zu c2) zueinander kann über einen relativ breiten Bereich varrieren. Zweckmäßig setzt sich die Komponente c) aus 80 - 95 Mol % c1) und 5 - 20 Mol % c2) zusammen, wobei sich die Mengenangaben zu c1) und c2) auf die Molverhältnisse der Oxide beziehen und c1) und c2) so auszuwählen sind, daß sie gemeinsam 100 Mol-% ergeben.

In bevorzugter Ausführungsform ist der Trägerkatalysator der Erfindung **dadurch gekennzeichnet**, daß das Oxidgemisch c) aus 80 - 90 Mol % c1) und 10 - 20 Mol% c2) besteht.

Weiters bevorzugt kennzeichnet sich der erfindungsgemäße Trägerkatalysator dadurch, daß das Oxidgemisch c) aus 88 - 90 Mol % c1) und 10 - 12 Mol % c2) besteht. Besonders vorteilhafte Trägerkatalysatoren ergeben sich im Rahmen der Erfindung, wenn das Trägermaterial d) mit 5 bis 6 Gew.-% einer Cerdioxid-Zirkondioxid-Mischung mit 10 bis 12 Mol % Zirkondioxid-Anteil versetzt ist.

Die Komponenten d) = Trägermaterial und c) = Oxidgemisch bilden zusammen den Träger des erfindungsgemäßen Trägerkatalysators.

Der erfindungsgemäße Trägerkatalysator weist als weiteren essentiellen Bestandteil a) 0,5 - 15 Gew.-%, bezogen auf metallisches Kupfer, einer oder mehrerer Kupfer-II-Verbindungen auf. Zu im Sinne der Erfindung einsetzbaren Kupfer-II-Verbindungen gehören unter anderem Kupferoxid, Kupfer(II)-Nitrat, - Sulfat, - Carbonat, - Halogenide wie z.B. - Bramid und/oder Kupfer-II-Chlorid.

Die Kupfer-II-Verbindungen sind entweder allein oder in Mischung von zwei oder mehreren im Trägerkatalysator der Erfindung enthalten. Bevorzugt von den genannten Kupfer-II-Verbindungen sind Kupfer-II-Halogenide. Ganz besonders bevorzugt ist Kupfer-II-chlorid. Bevorzugte Mengenbereich für die Komponente a) liegen im Bereich von etwa 1 - 12 Gew.-%, 2 - 14 Gew.-%, 3-13 Gew.-%, 4 - 8 Gew.-% sowie 5 - 7 Gew.-%, jeweils bezogen auf 100 Gew.-% der Summe von a) bis d), wobei die Komponente a) als Metall gerechnet ist.

Neben der Komponente a) muß der erfindungsgemäße Trägerkatalysator essentiell eine oder mehrere Alkalimetallverbindungen aufweisen. Einsetzbar sind alle ionischen Alkalimetallverbindungen. Zu den einsetzbaren Alkalimetallverbindungen für die Komponente b) gehören unter anderem die Halogenide, vorzugsweise Bromide und Chloride, Sulfate, Carbonate und Nitrate der Alkalimetalle. Insbesondere die Halogenide von Kalium, Natrium, Rubidium und/oder Cäsium. Vorzugsweise die Chloride von Kalium und/oder Natrium. Von besonderem Interesse sind Trägerkatalysatoren, worin Alkalimetallverbindung KCl ist.

Die Komponente b) ist im Trägerkatalysator der Erfindung in einer Menge von 0,1 bis 8 Gew.-% enthalten, wobei sich auch hier wie bei der Komponente a) die Mengenangabe auf das Alkalimetall bezieht. Bevorzugt sind Mengen im Bereich von 0,2 bis 5 Gew.-%, 0,4 - 3 Gew.-%, 0,5 bis 5 Gew.-%, 1 bis 4 Gew.-%. Besonderes zweckmäßig sind 1,5 bis 2,5 Gew.-%, jeweils gerechnet als Metall und immer bezogen auf das Gesamtgewicht der Komponenten a) bis d).

Besonders interessante erfindungsgemäße Trägerkatalysatoren zeichnen sich dadurch aus, daß das molare Verhältnis von $cu^{II}$-Verbindungen a) zu Alkalimetall-Verbindungen b) im Bereich von 1 : 1 bis 8 : 1 ist. Bevorzugt sind Verhältnisse im Bereich von 1 : 1 bis 6 : 1, ganz besonders vorteilig sind Verhältnisse im Bereich von 1,5 : 1 bis 5 : 1, von 2 : 1 bis 5 : 1 und von 2,5 : 1 bis 4 : 1. Bei der Oxichlorierung wurden die Reaktoren bewußt in verschiedene Zonen aufgeteilt, in denen Katalysatoren mit in Strömungsrichtung steigenden Aktivmassenbeladung zur Anwendung kommen.

In diesem Zusammenhang kann es bevorzugt sein, besondere Verhältnisse von a) : b) einzusetzen. Unter anderem ist es in einer ersten Variante bevorzugt, a) : b) - Verhältnisse von 1 : 1 bis etwa 1,5 : 1 einzusetzen.

Eine zweite Variante sieht vor, a) : b) - Verhältnisse zwischen 1,5 : 1 bis etwa 3 : 1 zu verwenden.

Noch eine Variante wird verwirklicht durch a) : b) - Verhältnisse im Bereich von 5 : 1 bis 8 : 1, während noch eine andere Variante auf a) : b) - Verhältnissen zwischen 3 : 1 mit etwa 5 : 1 benutzt wird.

Die Komponenten a) und b) bilden zusammen den Katalysator des erfindungsgemäßen Trägerkatalysators.

Per Träger des erfindungsgemäßen Trägerkatalysators weist ein bestimmtes Gesamtporenvolumen auf. Dieses liegt essentiell im Bereich von 0,65 bis 1,2 cm³/g. Liegt das Gesamtporenvolumen des Trägers c) + d) oder auch des Trägermaterials d) unterhalb von 0,65 oder oberhalb von 1,2 cm³/g lassen sich die weiter vorn beschriebenen mit der Erfindung erzielbaren vorteilhaften Effekte nicht in befriedigender Weise verwirklichen.

Besonders günstig mist das Trägermaterial d) oder der Träger c) + d) ein Gesamtporenvolumen im Bereich von 0,7 bis 1,0 cm³/g, vorteilhaft im Bereich von 0,7 bis 0,9 cm³/g auf.

Das Gesamtporenvolumen beträgt vorzugsweise 0,7 - 0,85 cm³/g. Für die Porenverteilung wird bevorzugt, daß 80 % des Gesamtporenvolumens durch Mesoporen mit einem Durchmesser von 4 bis 20 nm gebildet werden. Das Gesamtporenvolumen errechnet sich dabei aus der Summe der Mikro-, Meso- und Makroporenvolumina, wobei die Mikro- und Mesoporenvolumina durch Aufnahme von Stickstoffisothermen bzw. deren Auswertung nach BET, de Boer, Barret, Joyner und Halenda gemessen werden, während die Makroporenvolumina durch das Quecksilbereinpreßverfahren ermittelt werden.

Vorteilhafterweise besitzt der Träger, insbesondere der gamma-Aluminiumoxid-Träger, eine spezifische BET-Oberfläche von 150 bis 250 m²/g, vorzugsweise 180 bis 200 m²/g, wobei die BET-Oberfläche gemäß DIN 66131 bestimmt wird.

Die Geometrie des Trägerkatalysators ist mit entscheidend für den erfindungsgemäßen Erfolg. Im Rahmen der Erfindung liegen die Trägerkatalysatoren als zylindrische Hohlkörper mit wenigstens einem Durchtrittskanal vor, wobei für Durchmesser $d_a$ von bis zu 6 mm das Verhältnis von Höhe h zu Außendurchmesser $d_a$ kleiner 1,5 ist und für Durchmesser $d_a$ über 6 mm h : $d_a$ kleiner 0,6 sein muß.

Der niedrige Druckverlust der erfindungsgemäßen Katalysatorformlinge resultiert unter anderem aus deren geometrischen Abmessungen, wodurch eine extrem große freie Fläche im Querschnitt der Formkörper und/oder ein sehr hoher Lückengrad in der Katalysatorschüttung von etwa 0,4 bis 0,5 vorliegen.

Zu den im erfindungsgemäßen Sinne brauchbaren Geometrien gehören beispielsweise alle Hohlkörper, die durchströmbar sind, wie zylindrische Hohlkörper, ringförmige Hohlkörper, Hohlextrudate, ringförmige Hohlextrudate, Wagenräder mit 2 bis 12, vorzugsweise 4 bis 6, Speichen und/oder Monolithformkörper. Ein besonderer erfindungsgemäßer Trägerkatalysator liegt als zylindrisches Hohlextrudat, Wagenrad mit 2 bis 12, vorzugsweise 4 bis 6 Speichen und/oder Monolithformkörper vor.

Der Außendurchmesser der Trägerkatalysatorgeometrien kann über einen weiten Bereich varrieren. Beste Ergebnisse erzielt man grundsätzlich mit Trägerkatalysatoren, die dadurch gekennzeichnet sind, daß die Außendurchmesser im Bereich von 4 bis 10 mm sind.

Für den Fall, daß die Trägerkatalysatoren als zylindrische Hohlkörper in Form von Wagenrädern oder als Monolithformkörper vorliegen, ist es zweckmäßig, wenn das Verhältnis h/$d_a$ ≤ 0,6, bevorzugt ≤ 0,5 ist.

Für den Fall, daß die Trägerkatalysatoren als ringförmige Hohlextrudate vorliegen, ist ein h/$d_a$-Verhältnis von ≤ 1,5, vorzugsweise ≤ 1, zweckmäßig.

Die Vorteile der ringförmigen Hohlextrudate bestehen in vergleichsweise niedrigen Druckverlusten, niederen Schüttdichten und relativ großen äußeren Oberflächen pro Einheitsvolumen eines Reaktionsgefäßes. Die Vorteile der Wagenradgeometrie sind ebenfalls vergleichsweise niedrige Druckverluste und eine gleichmäßige Durchströmung der Speichenzwischenräume, die Erzeugung hoher Reaktionsgasturbulenzen im Inneren der Katalysatorformlinge sowie um die Formkörper herum durch Erhöhung der axialen und radialen Vermischung, das heißt Stofftransport entgegen der Strömungsrichtung, unter Einfluß eines vorhandenen Konzentrationsgradienten. Bei den Monolithformen liegen die Vorteile analog zu den wabenförmigen Monolithen in einer gleichmäßigen Durchströmung der an beiden Seiten offenen Kanäle in Längsrichtung, die die äußere Oberfläche des Katalysatorträgers pro Volumeneinheit stark erhöhen und in einer Steigerung des Diffusionsvermögens der gasförmigen Reaktanden in den Kanälen. Hohe Turbulenzen der Reaktionsgase an den Katalysatorformlingen und im Reaktionsraum verbessern bekanntermaßen die nachteiligen Wärmeabführungsprobleme, die aufgrund reduzierter Wärmeaustauschkoeffizienten zwischen den Festbett-Katalysatorformlingen bzw. zwischen den Katalysatorformlingen und dem Reaktionsgas auftreten. Träger mit großer freier Fläche im Querschnitt der Formlinge und/oder hohem Lückengrad in der Schüttung erzeugen bekanntlich einen niedrigen Widerstand gegenüber der Gasströmung, wodurch der Druckverlust über das Katalysatorbett bei vergleichbaren Schütthöhen geringer wird und somit erhöhte Raum-Zeit-Ausbeuten erzielt werden können. Ferner ist auch bekannt, daß ein großes Verhältnis von geometrischer Oberfläche zu Volumen des Formkörpers durch besseren Kontakt der Reaktionsgase mit der Katalysatoroberfläche die Aktivität bzw. den Umsatz erhöht, und die Diffusionsvorgänge der gasförmigen Edukte im Inneren der Katalysatorformlinge bzw. die Rückdiffusion der gasförmigen Produkte aus dem Inneren der Formkörper limitiert, womit die heterogen katalysierte Oxichlorierungs-Gasphasenreaktion, die an der leichter zugänglichen äußeren Katalysatoroberfläche bevorzugt und auch selktiver abläuft, effizienter gestaltet werden kann. Bekannt ist auch, daß Trägermaterialien mit relativ großem Gesamtporenvolumen und überwiegender Porenverteilung im Mikro- und Mesobereich die Aktivität steigern. Daneben ist es vorteilhaft, Trägerkatalysatoren mit einem möglichst niedrigen Schüttgewicht zu verwenden, wodurch pro Volumen vorgegebenem Reaktionsraum weniger Katalysatormasse erforderlich ist.

Obwohl die Katalysatoren mit den Merkmalen a) bis f) schon hervorragende Eigenschaften aufweisen, lassen sich erfindungsgemäße Trägerkatalysatoren weitens dadurch verbessern, daß sie zusätzlich einen Gehalt einer Yttrium-III-Verbindung aufweisen.

Die zusätzliche Aktivkomponente Yttrium-III-Verbindung kommt als Oxid oder Chlorid, vorzugsweise als Yttrium-III-chlorid, zum Einsatz.

Die als Temperaturstabilisatoren dienenden oxidischen Gemische aus Ceriterdenmetallen und Zirkon können entweder beim Aluminiumhydratfällprozeß in Hydroxidform mitgefällt werden oder nach der Formgebung und der erforderlichen Wärmbehandlung zusammen mit den Aktivkomponenten in Form löslicher Ceriterden- bzw. Zirkon-Verbindungen, beispielsweise als Acetate, aufgebracht werden, wobei diese Acetate beim abschließenden Calcinieren in die entsprechenden Oxide überführt werden.

Schon geringe Zusätze an Yttrium-III-Verbindung sind förderlich für die Leistung der erfindungsgemäßen Trägerkatalysatoren. Ein bevorzugter Trägerkatalysator der Erfindung ist dadurch gekennzeichnet, daß er 0,5 bis 10 Mol % Yttrium-III-Verbindung bezogen auf den molaren Gehalt an Kupfer-II-Verbindung(en), aufweist.

Weitens bevorzugt sind Gehalte im Bereich von 0,5 bis 5 Mol %, 1 bis 3 Mol %, insbesondere 1,5 - 2,5 Mol % Yttrium-III-Verbindung bezogen auf den molaren Gehalt an Kupfer-II-Verbindung.

Die Herstellung der Trägerkatalysatoren kann auf verschiedene Weise erfolgen.

Zum einen ist eine Variante bevorzugt, die dadurch gekennzeichnet ist, daß man

i) Trägermaterialien d) der Geometrie f) mit löslichen Precursoren der Komponenten c1) und c2) beaufschlagt;
ii) die Precursor-Verbindungen in die oxidische Form überführt; und
iii)die oxidisch beaufschlagten Trägermaterialen mit den Komponenten a) und b) belädt.

Alternativ dazu kann es auch bevorzugt sein, daß man

iv) eine Mischung aus den Komponenten c) und d) zur Geometrie f) formt; und
v) die geformten Trägermaterialien mit den Komponenten a) und b) belädt.

Beispielsweise kann man nach der Formgebung und einer Wärmebehandlung, die in einem oder mehreren Calcinierschritten erfolgen kann, wobei gefälltes Aluminiumoxidhydrat zusammen mit gegebenfalls mitgefällten Ceriterden- bzw. Zirkonoxid, die in das Aluminiumoxidgitter miteingebaut sind, dehydratisiert werden, den Träger mit den Aktivkomponenten tränken.

Alternativ kann durch primäres Tränken mit wässrigen Lösungen der Ceriterden-Zirkonsalze, anschließendes Calcinieren und nachfolgendes Tränken mit wässrigen Lösungen der Kupfer-, Alkalimetall- und gegebenenfalls Yttriumsalz der erfindungsgemäße Katalysator erhalten werden.

Alternativ dazu kann aber auch ein pulverförmiges Gemisch aus Aluminiumoxid, den drei Aktivkomponenten und den oxidischen Temperaturstabilisatoren verformt und calciniert werden.

Der erfindungsgemäße Katalysator kann bevorzugt in Festbett-Reaktoren eingesetzt werden.

Die Trägerkatalysatoren lassen sich sinnvoll in einer ganzen Reihe von Reaktionen einsetzen. Bevorzugt ist die Verwendung jedoch bei der Oxichlorierung von Ethylen.

Ein weiterer Gegenstand der Erfindung ist ein Verfahren zur Herstellung von 1,2-Dichlorethan (EDC) durch Oxichlorierung von Ethylen mit Chlorwasserstoff unter Verwendung des erfindungsgemäßen Trägerkatalysators. Als Oxidationsmittel dient Luft oder mit Sauerstoff angereicherte Luft, oder bevorzugt reiner Sauerstoff unter Kreisgasführung des überschüssigen, nicht umgesetzten Ethylens bei hochethylenhaltigem Kreisgas. Die Durchführung des Verfahrens kann einstufig oder in mehreren Stufen erfolgen. Bei der bevorzugten mehrstufigen Reaktionsführung können einzelne Einsatzstoffe, beispielsweise Chlorwasserstoff und/oder Luft/Sauerstoff aufgeteilt und jedem Reaktor getrennt zugeführt werden. Die Umsetzung kann auch in Kreisgasfahrweise durchgeführt werden, wobei das aus der Reaktionszone austretende Reaktionsgemisch mit frischem Chlorwasserstoff, Ethylen und Luft/Sauerstoff vermischt und in die Reaktionszone zurückgeführt wird. Aus dem Reaktionsgemisch wird vor der Rückführung 1,2-Dichlorethan und Reaktionswasser abgetrennt. Die Temperaturen in den Katalysatorschüttungen bewegen sich üblicherweise bei Werten zwischen 220°C und 300°C bei einem Druck von 3 bis 10 bar absolut. In Gegenwart der erfindungsgemäßen Trägerkatalysatoren genügen 4 bis 8 % Ethylen - bzw. 5 bis 10 % Sauerstoffüberschuß, jeweils bezogen auf die Stöchiometrie zu Chlorwasserstoff bei Luftfahrweise, um den Chlorwasserstoffumsatz bei einer extrem hohen Chlorwasserstoff-Beschickung von 1,4 bis 2 Nm$^3$ pro Liter Katalysatorvolumen und Stunde nahezu quantitativ zu gestalten.

Bei der Durchführung des Oxichlorierungsverfahrens unter Verwendung des Trägerkatalysators ist es zweckmäßig, die Aktivität des Katalysators abzustufen, so daß die Aktivität im Reaktor bei einstufiger Fahrweise bzw. bei mehrstufiger Fahrweise zumindest in der ersten und zweiten Stufe in Produktstromrichtung zunimmt. Unter Vermeidung der insgesamt reaktionsschädlichen Verdünnung des Katalysators mit Inertmaterial ist es bevorzugt, Kupfer-II-clorid-Konzentrationen abgestuft zwischen 7,5 und 22,5 Gew.-% bezogen auf das Gesamtgewicht des Trägerkatalysators, zu verwenden, wobei die Alkalimetallchloridzugabe in molarem Verhältnis von Kupfer-II-chlorid zu Alkalimetallchlorid ebenfalls abgestuft bei Werten zwischen 1 : 1 und 6 : 1 sowie die Yttrium-III-chlorid-Dotierung bei Werten zwischen 0,5 und 5 Mol %, bezogen auf den jeweiligen molaren Gehalt an Kupfer-II-chlorid, liegt.

Nachfolgend wird die Erfindung durch Ausführbeispiele unter Bezugnahme auf die beigefügten Figuren näher erläutert.

In den Figuren zeigen

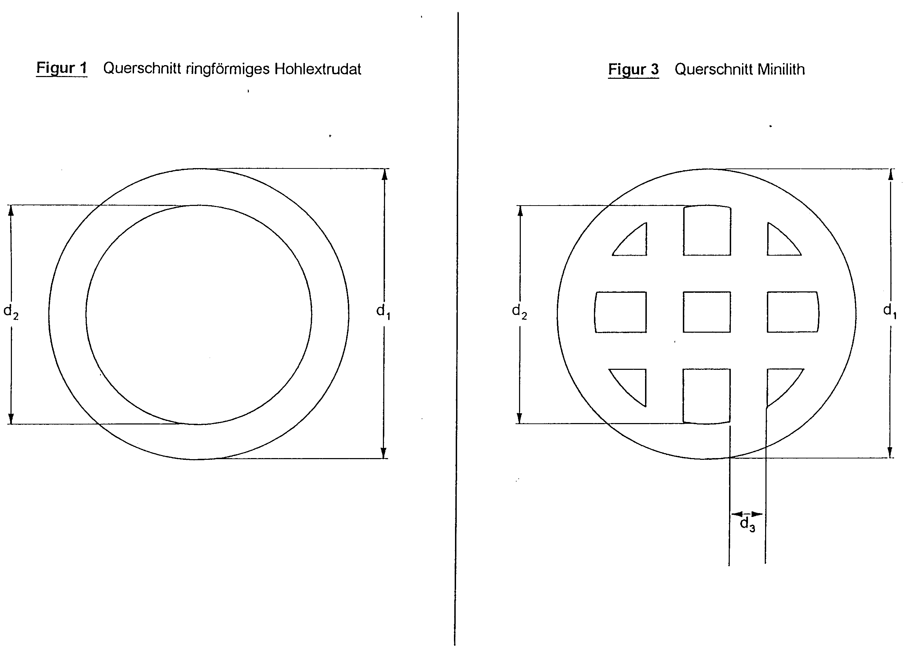

Figur 1      ein ringförmiges Hohlextrudat im Querschnitt;

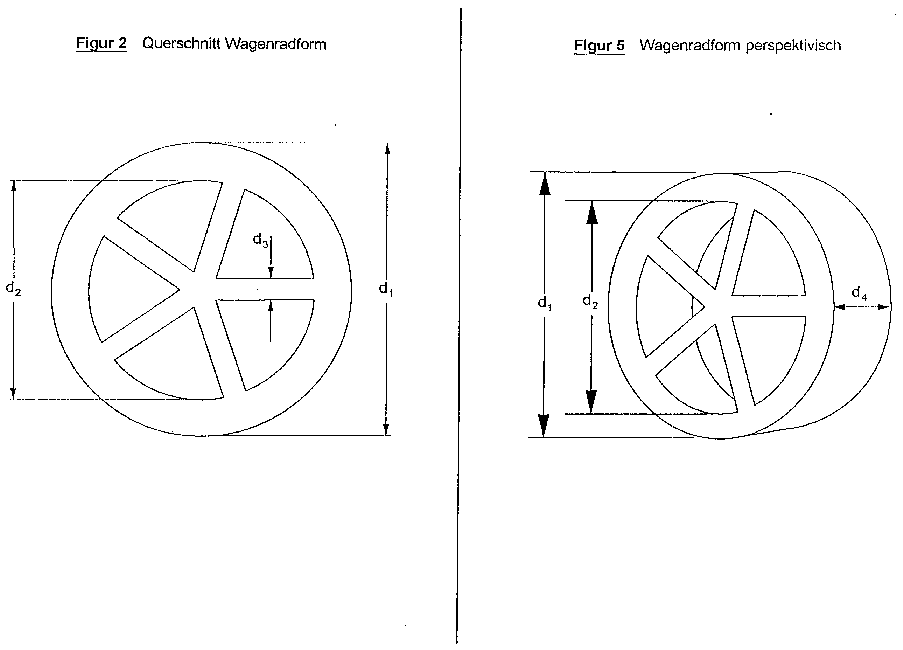

Figur 2      einen Querschnitt durch einen Trägerkatalysator in Wagenradform;

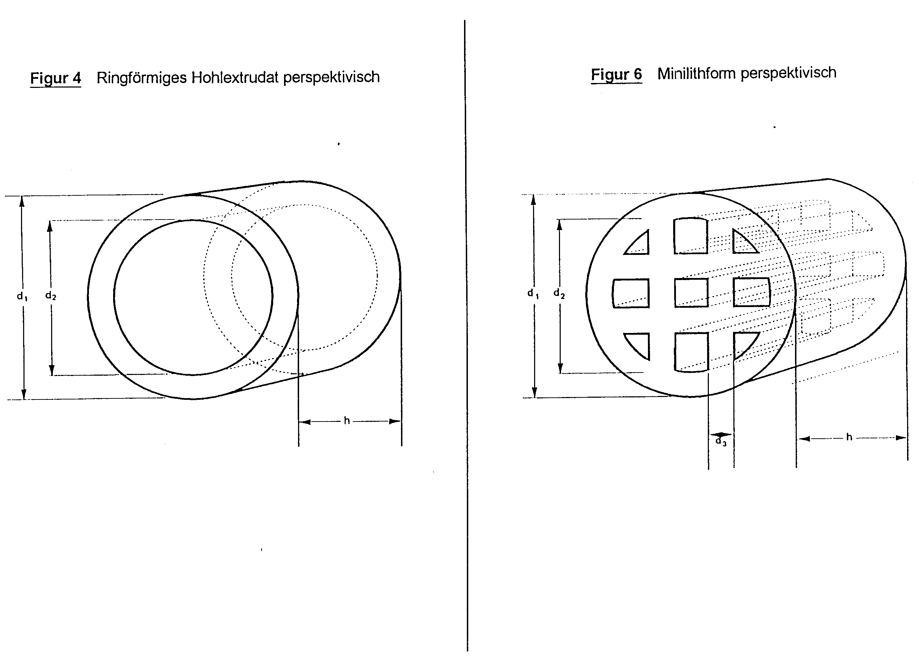

Figur 3      einen Trägerkatalysator in Monodithform im Querschnitt;
Figur 4      das ringförmige Hohlextrudat aus Figur 1 in perspektivischer Darstellung;
Figur 5      eine perspektivische Darstellung der Wagenradform aus Figur 2; und
Figur 6      die perspektivische Darstellung der Mandithform aus Figur 3;
Figur 7      eine Prinzipskizze für ein Verfahren zur Oxichlorierung.

Alle Figuren betreffen erfindungsgemäße Ausführungsformen.

Vorzugsweise beträgt der Aussendurchmesser $d_1$ der Wagenräder und monolithischen Formkörper 8 bis 10 mm. Für den Innendurchmesser $d_2$ wird bei den Hohlextrudaten der Bereich von 1 bis 2 mm bzw. bei den Wagenrädern und monolithischen Formkörper der Bereich von 6 bis 8 mm bevorzugt. Die Speichenstärke bzw. Stegstärke $d_3$ beträgt bei den Wagenrädern bzw. monolithischen Formkörper vorzugsweise 1 bis 1,5 mm. Für die Höhe h der Formlinge wird der Bereich von 3 bis 5 mm bevorzugt. Der äquivalente Durchmesser $d_{\ddot{a}}$ berechnet sich nach der Formel $d_{\ddot{a}} = 2r$, wobei die Beziehung $\gamma = (V/\pi \cdot h)^{1/2}$ mit V = Volumen des Formkörpers gilt.

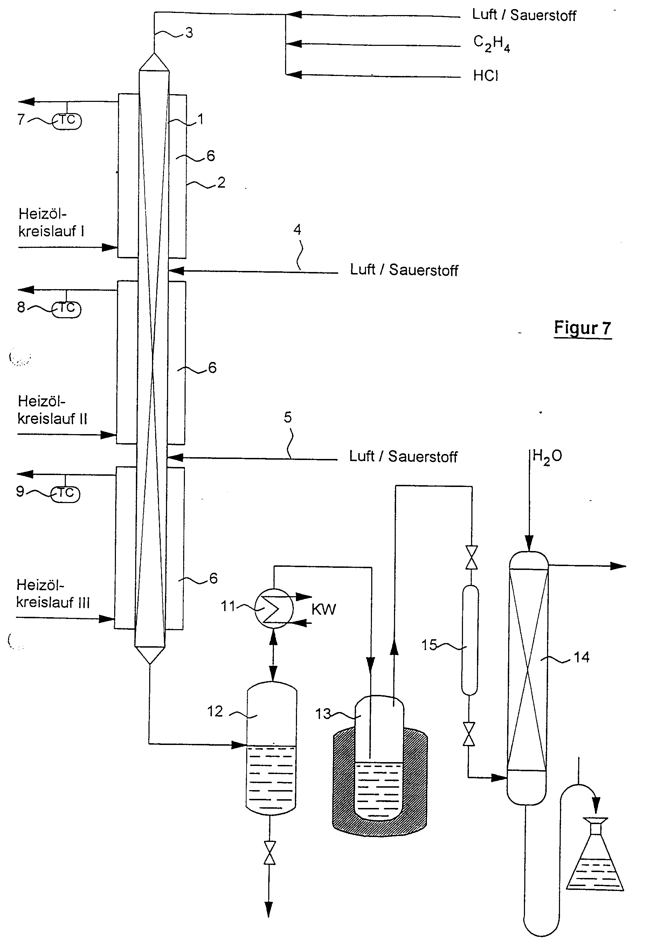

Die Erfindung wird durch die nachstehend beschriebenen Beispiele weiter erläutert, wobei auch auf Figur 7 Bezug genommen wird.

Beispiel 1

Es werden die in Tabelle 1 aufgelistete Trägermaterialien verwendet. Diese Trägermaterialien sind in 150 x 150 mm Nickelrahmen mit beidseitiger gasdurchlässiger Nickeldrahtgeflecht-Belegung in einer Schütthöhe von jeweils 50 mm eingefüllt. Die mit den jeweiligen Trägermaterialien befüllten Nickeltaschen werden in die obere, mit Keramik-Sattelkörpern aufgefüllte Haube des zweiten von insgesamt drei in Serie geschalteten Oxichlorierungsreaktoren derart eingebracht, daß der 230-grädige Gasstrom aus dem Unterteil des vorgeschalteten Oxichlorierungsreaktors 1 - im wesentlichen bestehend aus den dampfförmigen Reaktionsprodukten 1,2-Dichlorethan und Wasser sowie nichtumgesetztem Chlorwasserstoff, Ethylen, Sauerstoff bzw. Stickstoff aus der dem 1. Reaktor aufgegebenen Luft und aus Luft für Reaktor 2 - die Taschen ungekühlt durchströmt. Da das Trägermaterial keine Aktivkomponente enthält, findet keine Oxichlorierungsreaktion statt, so daß die Wärmeeinwirkung auf das Trägermaterial nahezu isotherm verläuft.

Tabelle 1

Charakteristische Daten der in Beispiel 1 verwendeten geformten Katalysatorträger auf gamma-Aluminiumoxid-Basis

| Katalysator-Bezeichnung | Katalysator-Form | Außen-durch-messer (mm) | Innen-durch-messer (mm) | Höhe h (mm) | Spei-chen- bzw. Steg-stärke (mm) | Volumen des Trägers (mm³) | $d_a$ (mm) | $h/d_a$ | Poren-Volumen (cm³/g) | Temperatur-stabilisator (Gew% bezogen auf $\gamma$-Al$_2$O$_3$) |
|---|---|---|---|---|---|---|---|---|---|---|
| A | Hohlzylinder | 4,5 | 1,5 | 4 | - | 56,6 | 4,24 | 0,94 | 0,8 | - |
| B | Wagenrad | 8,5 | 6,3 | 4 | 1,2 | 170 | 7,36 | 0,54 | 0,8 | - |
| C | Minilith | 8,5 | 6,5 | 4 | 1,0 | 177 | 7,51 | 0,53 | 0,8 | - |
| D | Hohlzylinder | 5 | 1,5 | 10 | - | 179 | 4,77 | 2,10 | 0,6 | 5,5 % La$_2$O$_3$ |
| E | Hohlzylinder | 5 | 1,5 | 4 | - | 71,5 | 4,77 | 0,84 | 0,6 | 5,5 % Sm$_2$O$_3$ |
| F | Hohlzylinder | 5 | 1,5 | 10 | - | 179 | 4,77 | 2,10 | 0,6 | 5,5 % CeO$_2$ |
| G | Hohlzylinder | 5 | 1,5 | 10 | - | 179 | 4,77 | 2,10 | 0,6 | 5,5 % ZrO$_2$ |
| H | Hohlzylinder | 5 | 1,5 | 10 | - | 179 | 4,77 | 2,10 | 0,6 | 5,0 % CeO$_2$ + 0,5 % ZrO$_2$ |
| J | Wagenrad | 8 | 4,8 | 8 | 1,6 | 400 | 7,98 | 1,00 | 0,5 | 7,0 % La$_2$O$_3$ |
| K | Wagenrad | 8,5 | 6,3 | 4 | 1,2 | 170 | 7,36 | 0,34 | 0,5 | 10,0 % CeO$_2$ |
| L | Wagenrad | 8,5 | 4,8 | 8 | 1,6 | 400 | 7,98 | 1,00 | 0,5 | 5,0 % CeO$_2$ + 0,5 % ZrO$_2$ |
| M | Monolithform | 8,5 | 6,5 | 8 | 1,0 | 354 | 7,51 | 1,10 | 0,6 | 4,0 % Sm$_2$O$_3$ |
| N | Monolithform | 8,5 | 6,3 | 4 | 1,2 | 170 | 7,36 | 0,54 | 0,6 | 10 % ZrO$_2$ |
| O | Monolithform | 8,5 | 6,3 | 4 | 1,2 | 170 | 7,36 | 0,54 | 0,6 | 7,0 % CeO$_2$ + 1,0 % ZrO$_2$ |
| P | Säule | 5 | - | 5 | - | 98,2 | 5,00 | 1,00 | 0,8 | 5,0 % CeO$_2$ + 0,5 % ZrO$_2$ |
| Q | Kugeln | 4 | - | 4 | - | 33,5 | 3,27 | 1,22 | 0,8 | 9,0 % CeO$_2$ + 1,6 % ZrO$_2$ |

Die Bruchhärte der einzelnen Trägermaterialien (ausgedrückt in Newton N) wurde vor der Wärmebehandlung bestimmt. Nach einjähriger Wärmeeinwirkung unter hydrothermalen Bedingungen bei 230°C und einem Druck von 6,5 bar absolut werden die Probetaschen ausgebaut und erneut die Bruchhärte der einzelnen Trägerproben gemessen, wobei die Abnahme der Bruchhärte ein Maß für die Temperaturstabilität der Trägermaterialien darstellt.

Die Ergebnisse dieser Temperaturstabilitätsuntersuchungen sind in Tabelle 2 festgehalten. Daraus ist ersichtlich, daß Träger der beanspruchten geometrischen Formgebung ohne Zusatz von Temperaturstabilisatoren und unabhängig vom $h/d_ä$-Wert wie von der Porosität am wenigsten temperaturbeständig sind bzw. der Zusatz von Zirkondioxid keinen erkennbaren temperaturstabilisierenden Effekt erbringt (Träger A, B, C bzw. Träger G,N).

Die literaturbekannten Temperaturstabilisatoren, wie die Oxide von Lanthan, Cer und Samarium bewirken unter vergleichbaren Bedingungen nur eine mäßige Wärmebeständigkeit des Trägermaterials gamma-Aluminiumoxid (Träger D,E,F,J,K,M), während der erfindungsgemäße Temperaturstabilisator Cerdioxid/Zirkondioxid dem gamma-Aluminiumoxid die beste Wärmefestigkeit verleiht (Träger H,L,O), wobei bei Verwendung der beanspruchten Formkörper weder eine Abhängigkeit vom $h/d_ä$-Wert noch von der Porosität erkennbar ist. Überraschend ist jedoch, daß der bevorzugte erfindungsgemäße Thermostabilisator Cerdioxid/Zirkondioxid bei säulen- und kugelförmigen Trägern nicht einmal die Wirkung der Lanthanerdenoxide erreicht (Träger P, Q).

Dies kann - ohne Anspruch auf Richtigkeit des Erklärungsversuches zu erheben - dafür sprechen, daß die Wärmefestigkeit nicht - wie für die mechanische Festigkeit allgemein angenommen - von der Dicke der Formkörper bzw. deren Formelemente abhängt. Vielmehr unterliegt sie anderen Gesetzen, wie z.B. einer vorteilhaften Wärmeabführung infolge Geometrie/Wärmeleitfähigkeit.

Zusammenfassend ergibt sich folgende releative prozentuale Wärmefestigkeit der beanspruchten Formkörper Hohlzylinder-, Wagenrad- und Minilithformen in nachstehender Abstufung

| | |
|---|---|
| gamma-Aluminiumoxid + Cer-/Zirkondioxid | 100 % |
| gamma-Aluminiumoxid + Lanthanoxid | 57 % |
| oder Cerdioxid | |
| oder Samariumoxid | |
| gamma-Aluminiumoxid + Zirkonoxid bzw. ohne Thermostabilisator | 38 % |

Säulen- bzw. kugelförmige Träger mit Cerdioxid/Zirkondioxid-Zuschlag weisen dagegen nur die halbe Wärmefestigkeit bezogen auf die beanspruchten Trägerformen mit Cerdioxid/Zirkondioxidzugabe auf.

Tabelle 2

| Beständigkeitsbeurteilung von Trägermaterialien gemäß Tabelle 1 aufgrund der Veränderung der Bruchhärte | | | |
|---|---|---|---|
| Trägermaterial | Bruchhärte (in N) | | Verlust an Bruchhärte durch Wärmeeinwirkung (in %) |
| | vorher | nachher | |
| A | 23 | 10 | 57 |
| B | 44 | 22 | 50 |
| C | 36 | 17 | 53 |
| D | 26 | 16 | 38 |
| E | 23 | 15 | 35 |
| F | 26 | 15 | 42 |
| G | 26 | 12 | 54 |
| H | 26 | 20 | 23 |
| J | 44 | 29 | 34 |

Tabelle 2 (fortgesetzt)

| Beständigkeitsbeurteilung von Trägermaterialien gemäß Tabelle 1 aufgrund der Veränderung der Bruchhärte | | | |
|---|---|---|---|
| Trägermaterial | Bruchhärte (in N) | | Verlust an Bruchhärte durch Wärmeeinwirkung (in %) |
| | vorher | nachher | |
| K | 44 | 30 | 32 |
| L | 44 | 36 | 18 |
| M | 40 | 27 | 32 |
| N | 40 | 19 | 53 |
| O | 40 | 32 | 20 |
| P | 55 | 33 | 40 |
| Q | 60 | 35 | 42 |

Beispiel 1a:

Trägermaterialien der beanspruchten geometrischen Formgebung und Ce/Zr-Dotierung werden durch Tränken von jeweils 4000 g $\gamma$-Aluminiumoxid in Hohlzylinder- bzw. Wagenrad- bzw. Minilithform mit einer wässrigen, essigsauren Lösung von Ce(II)acetat und Zirkonium(II)acetat gemäß den vorgegebenen Dotierungsanteilen von 5 % Ce und 0,5 % Zr bezogen auf das Trägergewicht imprägniert, wobei die Menge der wässrigen Lösung so bemessen ist, daß die gesamte Lösung von $\gamma$-Aluminiumoxid aufgesogen wird. Anschließend werden die imprägnierten Formkörper bei 550°C im Luftstrom getempert, wobei unter Umwandlung der Imprägniersalze in oxidische Spezies eine Entwässerung erfolgt und trägermaterialien der beanspruchten geometrischen Form mit folgender Zusammensetzung resultieren:

94,5 Gew.-% $Al_2O_3$
5,0 Gew.-% $CeO_2$
0,5 Gew.-% $ZrO_2$

Aus diesen temperaturstabilisierten Trägern werden Trägerkatalysatoren der beanspruchten Formgebung/Dotierung in vier verschiedenen Formulierungen hergestellt, die dem Beladungsmuster des dreistufigen Oxychlorierungsreaktors Rechnung tragen (Beispiel III in Tabelle Ia)

Typ A

300 g temperaturstabilisierter Träger werden mit einer wässrigen Lösung von 33,91 g CUCl2 x 2 H2O und 13,44 g KCl imprägniert und bei 135°C getrocknet. Chemische Zusammensetzung des Katalysators Typ A nach Trocknung:

83,31 Gew.-% $Al_2O_3$
4,4 Gew.-% $CeO_2$
0,44 Gew.-% $ZrO_2$
7,9 Gew.-% $CuCl_2$
3,95 Gew.-% KCl

Typ B

300 g temperaturstabilisierter Träger werden mit einer wässrigen Lösung von 54,08 g $CuCl_2$ x 2H2O und 12,8 g KCl imprägniert und bei 135°C getrocknet. Chemische Zusammensetzung des Trägerkatalysators Typ B nach Trocknung.

79,76 Gew.-% $Al_2O_3$
4,22 Gew.-% $CeO_2$

0,42 Gew.-% $ZrO_2$
12,00 Gew.-% $CuCl_2$
3,60 Gew.-% KCl

Typ C

300 g temperaturstabilisierter Träger werden mit einer wässrigen Lösung von 113,52 g $CuCl_2$ x 2H2O und 8,36 g KCl imprägniert und bei 135°C getrocknet. Chemische Zusammensetzung des Trägerkatalysators Typ C nach Trocknung:

71,26 Gew.-% $Al_2O_3$
3,76 Gew.-% $CeO_2$
0,38 Gew.-% $ZrO_2$
22,50 Gew.-% $CuCl_2$
2,10 Gew.-% KCl

Typ D

300 g temperaturstabilisiertes Trägermaterial werden mit einer wässrigen Lösung von 115,46 g $CuCl_2$ x 2$H_2O$ und 15,25 g KCl imprägniert und bei 135°C getrocknet. Chemische Zusammensetzung des Trägerkatalysators Typ D nach Trocknung:

69,77 Gew.-% $Al_2O_3$
3,69 Gew.-% $CeO_2$
0,37 Gew.-% $ZrO_2$
22,50 Gew.-% $CuCl_2$
3,75 Gew.-% KCl

Als Referenzkatalysatoren wurden Katalysatoren folgender Zusammensetzung verwendet, die gemäß den zuvor beschriebenen Imprägniermethodik mittels wässriger Lösung hergestellt werden. Hierbei wurden jedoch nicht temperaturstabilisierte $\gamma$-$Al_2O_3$ Trägermaterialien verwendet.
Chem. Zusammensetzung der Katalysatoren gemäß Beispiel I in Tabelle I a

Typ A:7,9 Gew.-% $CuCl_2$; 3,95 Gew.-% KCl; 88,15 Gew.-% $Al_2O_3$
Typ B:12,0 Gew.-% $CuCl_2$; 3,60 Gew.- % KCl; 84,40 Gew.- % $Al_2O_3$
Typ C:22,5 Gew.-% $CuCl_2$; 2,10 Gew.-% KCl; 75,4 Gew.-% $Al_2O_3$
Typ D:22,5 Gew.-% $CuCl_2$; 3,75 Gew.-% KCl; 73,75 Gew.-% $Al_2O_3$

Die Katalysatoren gemäß Beispiel II in Tabelle Ia wurden zusätzlich unter Zusatz von $YCl_3$ x 6 $H_2O$ imprägniert. Sie haben nach der Trocknung folgende chemische Zusammensetzung:

Typ A: 7,9 Gew.-% $CuCl_2$; 3,95 Gew.-% KCl, 0,12 Gew.-% $YCl_3$, 88,03 Gew.-% $Al_2O_3$
Typ B: 12,0 Gew.-% $CuCl_2$, 3,6 Gew.-% KCl; 0,44 Gew.-% $YCl_3$; 83,96 Gew.-% $Al_2O_3$
Typ C: 22,5 Gew.-% $CuCl_2$; 2,1 Gew.-% KCl; 0,82 Gew.-% $YCl_3$; 74,58 Gew.-% $Al_2O_3$
Typ D: 22,5 Gew.-% $CuCl_2$; 3,75 Gew.-% KCl; 0,82 Gew.-% $YCl_3$; 72,93 Gew.-% $Al_2O_3$

Im Rohre des ersten Reaktors einer Luft-Oxychlorierungsanlage mit Dreireaktortechnologie (ausführliche Beschreibung in Beispiel 2) wurden die beschriebenen Katalysatoren nach der entsprechenden Beladungsabstufung eingebracht. Der Ausbau der Katalysatoren erfolgte nach 12 Monaten.
Die visuelle Beurteilung der Ausbaukatalysatoren gemäß Tabelle IIa zeigt eindeutig die Temperatur-/Formstabilisierende Wirkung der Ce/Zr-Dotierung. Die nicht temperaturstabilisierten Katalysatoren gemäß Beispiel I und II zerfielen in den Zonen 2 - 4 zu Staub. Für mit Ce/Zr-Oxiden präparierte Katalysatoren war über den gesamten Zonenbereich bei vergleichbarer Betriebszeit nur ein Zerfall in Grob- und Feinbruch gegeben. Ein Zusatz von Yttriumchlorid alleine bewirkt keine Temperatur-/Formstabilisierung.

Tabelle I a

| Katalysatoren für die Ermittlung der Wärmebeständigkeit gemäß Beispiel 1a unter den Betriebsbedingungen des ersten Reaktors einer Luft-Oxichlorierung mit Dreireaktorentechnik | | | | | | |
|---|---|---|---|---|---|---|
| Katalysatorbe-zeichnung | Katalysatorform | Außendurch-messer [mm] | Innendurch-messer [mm] | Höhe [mm] | Porenvolumen [ml/g] | Zusätze [Gew%] bezogen auf Träger |
| I | Hohlzylinder | 4,5 | 1,5 | 7-15 | 0,6 | kein Zusatz |
| II | Wagonwheel | 8,5 | 6,3 | 5 | 0,6 | Zusatz von $YI_3$ typabhängig |
| III | Wagonwheel | 8,5 | 6,3 | 5 | 0,6 | 5 Gew% $CeO_2$ 0,5 Gew% $ZrO_2$ |

Tabelle II a

| Wärmebeständigkeit der Katalysatoren gemäß Tabelle I und Beispiel 1a unter den Betriebsbedingungen des ersten Reaktors einer Luft-Oxichlorierung mit Dreireaktorentechnik -Visuelle Beurteilung nach Ausbau (12 Monate Betriebszeit)- | | | | | |
|---|---|---|---|---|---|
| Katalysator | Reaktorzone 1 | Reaktorzone 2 | Reaktorzone 3 | Reaktorzone 4 | Reaktorzone 5 |
| Typen | Typ A | Typ A | Typ B | Typ C | Typ C |
| I | Grob- und Feinbruch | Feinbruch hoher Staubanteil | Feinstaub | Feinstaub | Grobbruch |
| II Schüttgewicht [g/l] | Grob-und Fein bruch 855 | Feinstaub 1110 | Feinstaub 1030 | Feinstaub 1060 | Grob- und Feinbruch 850 |
| III Schüttgewicht [g/l] | Grob-und Feinbruch 890 | | | | |

Beispiel 2

Trägermaterialien der beanspruchten geometrischen Formgebung werden durch Tränken von jeweils 4000 g gamma-Aluminiumoxid in Hohlzylinder- bzw. Wagenrad- bzw. Minilithform mit den in Tabelle 3 aufgelisteten charakteristischen Daten mit einer wässrigen Lösung von 477,5 g Cer-IV-nitrat und 39,7 g Zirkonyldinitrat imprägniert, wobei die Menge der wässrigen Lösung so bemessen ist, daß die gesamte Lösung von gamma-Aluminiumoxid aufgesogen wird. Anschließend werden die imprägnierten Formkörper bei 150°C im Luftstrom getempert, wobei unter Entweichen von Sauerstoff und nitrosen Gasen aus der Nitratsetzung eine Entwässerung erfolgt und Trägermaterialien der beanspruchten geometrischen Formgebung mit folgender chemischer Zusammensetzung resultieren:

94,5 Gew.-% $\gamma$-$Al_2O_3$
5,0 Gew.-% $CeO_2$
0,5 Gew.-% $ZrO_2$

Aus diesem temperaturstabilisiertem Trägermaterial werden Trägerkatalysatoren der beanspruchten Formgebung gemäß Tabelle 3 mit den Aktivkomponenten gemäß Tabelle 4 beladen, wobei jeder der mit R bis Z bezeichneten Trägerkatalysatoren in den Typen A,B,C,D hergestellt wird.

Typ A:

Jeweils 300 g temperaturstabilisierter Trägerkatalysator werden mit einer wässrigen Lösung von 34,19 g

$CuCl_2 \cdot 2H_2O$, 13,49 g KCl und 1,54 g $YCl_3 \cdot 6H_2O$ imprägniert und bei 135°C getrocknet.
Chemische Zusammensetzung des Trägerkatalysators Typ A nach Trocknung:

83,02 Gew.-% $Al_2O_3$
4,4 Gew.-% $CeO_2$
0,44 Gew.-% $ZrO_2$
7,9 Gew.-% $CuCl_2$
3,95 Gew.-% KCl
0,29 Gew.-% $YCl_3$

Typ B:

Jeweils 300 g temperaturstabilisiertes Trägermaterial werden mit einer wässrigen Lösung von 54,36 g $CuCl_2 \cdot 2H_2O$, 12,86 g KCl und 2,44 g $Ycl_3 \cdot 6H_2O$ imprägniert und bei 135°C getrocknet. Chemische Zusammensetzung des Trägerkatalysators Typ B nach Trocknung:

79,34 Gew% $Al_2O_3$
4,20 Gew% $CeO_2$
0,42 Gew% $ZrO_2$
12,00 Gew% $CuCl_2$
3,60 Gew% KCl
0,44 Gew% $YCl_3$

Typ C

Jeweils 300 g temperaturstabilisiertes Trägermaterial werden mit einer wässrigen Lösung von 114,73 g $CuCl_2 \cdot 2H_2O$, 8,45 g KCl und 5,13 g $YCl_3 \cdot 6H_2O$ imprägniert und bei 135°C getrocknet.
Chemische Zusammensetzung des Trägerkatalysators Typ C nach Trocknung:

70,48 Gew.-% $Al_2O_3$
3,73 Gew.-% $CeO_2$
0,37 Gew.-% $ZrO_2$
22,50 Gew.-% $CuCl_2$
2,10 Gew.-% KCl
0,82 Gew.-% $YCl_3$

Typ D:

Jeweils 300 g temperaturstabilisiertes Trägermaterial werden mit einer wässrigen Lösung von 117,33 g $CuCl_2 \cdot 2H_2O$, 15,43 g KCl und 5,23 g $YCl_3 \cdot 6H_2O$ imprägniert und bei 135°C getrocknet.
Chemische Zusammensetzung des Trägerkatalysators Typ D nach Trocknung:

68,92 Gew.-% $Al_2O_3$
3,65 Gew.-% $CeO_2$
0,36 Gew.-% $ZrO_2$
22,50 Gew.-% $CuCl_2$
3,75 Gew.-% KCl
0,82 Gew.-% $YCl_3$

Tabelle 3:

Charaktaristische Daten der in Beispiel 2 verwendeten Trägerkatalysatoren auf Basis gamma-Aluminiumoxid, vermischt mit 5 Gew.-% Cerdioxid und 0,5 Gew.-% Zirkondioxid, jeweils bezogen auf gamma-Aluminiumdioxid

| Kataly-sator-Bezeich-nung | Katalysator-Form | Außen-durch-messer | Innen-durch-messer (mm) | Höhe h (mm) | Speichen- bzw. Steg-stärke (mm) | Volumen des Körpers $(mm^3)$ | $d_{\ddot{a}}$ (mm) | $h/d_{\ddot{a}}$ | Poren-Volumen $(cm^3/g)$ | Aktiv-komponenten anteil |
|---|---|---|---|---|---|---|---|---|---|---|
| R | Wagenrad | 8,5 | 6,3 | 4 | 1,2 | 170 | 7,36 | 0,54 | 0,8 | |
| S | Wagenrad | 8,5 | 6,3 | 4 | 1,2 | 170 | 7,36 | 0,54 | 0,5 | |
| T | Wagenrad | 8,5 | 4,8 | 8 | 1,2 | 400 | 7,98 | 1,00 | 0,8 | siehe |
| U | Hohlzylinder | 4,5 | 1,5 | 4 | - | 56,6 | 4,24 | 0,94 | 0,8 | Tabelle 4 |
| V | Hohlzylinder | 5,0 | 1,5 | 4 | - | 71,5 | 4,77 | 0,84 | 0,6 | |
| W | Hohlzylinder | 5,0 | 1,5 | 10 | - | 179 | 4,77 | 2,10 | 0,8 | |
| X | Monolithform | 8,5 | 6,3 | 4 | 1,2 | 170 | 7,36 | 0,54 | 0,8 | |
| Y | Monolithform | 8,5 | 6,3 | 4 | 1,2 | 170 | 7,36 | 0,54 | 0,6 | |
| Z | Monolithform | 8,5 | 6,5 | 8 | 1,0 | 354 | 7,51 | 1,10 | 0,8 | |

Tabelle 4

| Aktivkomponentenanteile der in Tabelle 3 aufgelisteten Trägerkatalysatoren und Reaktorrohrfüllplan | | | |
|---|---|---|---|
| Von jedem der mit R bis Z bezeichneten Trägerkatalysatoren gemäß Tabelle 3 kommen jeweils folgende Untertypen zum Einsatz (Zahlenangaben in Gew.-% jeweils bezogen auf das Gesamtgewicht des Trägerkatalysators). | | | |
| | $CuCl_2$ | KCl | $YI_3$ |
| Typ A | 7,9 | 3,95 | 0,29 |
| Typ B | 12,0 | 3,60 | 0,44 |
| Typ C | 22,5 | 2,10 | 0,82 |
| Typ D | 22,5 | 3,75 | 0,82 |

Der Reaktorrohrfüllplan im Ersten von drei in Serie geschalteten Oxichlorierungsreaktoren, in dem jeweils 9 von insgesamt 3200 Rohren mit den Trägerkatalysatoren R bis Z gemäß Tabelle 3 gleichmäßig verteilt über den Reaktorquerschnitt beschickt waren, lautet (von oben nach unten, das heißt in Produktstromrichtung):

| 1. Zone | Typ A | von Katalysator R...Z |
|---|---|---|
| 2. Zone | Typ A | von Katalysator R...Z |
| 3. Zone | Typ B | von Katalysator R...Z |
| 4. Zone | Typ C | von Katalysator R...Z |
| 5. Zone | Typ D | von Katalysator R...Z |

In den ersten Reaktor einer Luft-Oxichlorierungsanlage mit Dreireaktortechnologie, wobei die Reaktoren durch Heißwasserverdampfung gekühlt werden, wird ein frischer Trägerkatalysator auf Basis gamma-Aluminiumoxid ohne Thermostabilisatorzusatz in Hohlzylinderform mit den Abmessungen gemäß EP 0 461 431 eingefüllt, der mit Kupferchlorid und Kaliumchlorid in den 4 Typen A bis D gemäß Tabelle 4 - jedoch ohne Yttrium-III-chlorid-Zusatzimprägniert ist, wobei der in Tabelle 4 beschriebene zonenförmige Füllplan verwendet wird. 9 der insgesamt 3200 Reaktorrohre werden jedoch gleichmäßig verteilt über den Reaktorquerschnitt mit den Katalysatorproben R bis Z gemäß Tabelle 3 bzw. Tabelle 4 befüllt.
Die Reaktoren 2 und 3 enthalten schon gebrauchte, aber noch intakte Katalysatoren gemäß EP 0 461 431.
Per Reaktor 1 wird mit 400 kmol/h HCl, 220 Kmol/h Ethylen und 219 kmol/h Luft beaufschlagt. Weitere 219 kmol/h Luft bzw. 109 kmol/h Luft werden dem Austrittsgemisch von Reaktor 1 bzw. von Reaktor 2 zugegeben.
Bei einem Systemdruck von 6,5 bar absolut, gemessen am Eintritt zu Reaktor 1, und einem Dampftrommel- bzw. Reaktorkühlmanteldruck von 20 bar absolut werden die Edukte zu 1,2-Dichlorethan und Wasser umgesetzt, wobei der mit der Luft zum Reaktor 1 zugesetzte Sauerstoff nahezu quantitativ umgesetzt wird. Überschüssiges HCl und Ethylen werden zusammen mit den Reaktionsprodukten unter weiterer Zugabe von Luft in den nachfolgenden Reaktoren in 1,2-Dichlorethan und Wasser umgewandelt. Nach 8 Monaten Laufzeit, in deren Verlauf der Dampftrommeldruck von Reaktor 1 sukzessive bis auf einen Wert von 29 bar absolut angehoben werden mußte, um die Höhe und Lage der Hot-Spot-Temperaturen zu regulieren bzw. um den allmählich voranschreitenden Aktivitätsverlust zu kompensieren kam es bei steigendem Verlust an Reaktionsselektivität zu einer plötzlichen Bettverdichtung im ersten Reaktor, sodaß die Anlage abgestellt und ein Katalysatorwechsel vorgenommen werden mußte.
Bei Entleeren zeigte sich, daß die kommerziellen Katalysatoren gemäß EP 0 461 431 in allen Zonen nahezu in Staub bzw. grob- und feinkörnigen Bruch zerfallen waren und überwiegend die alpha-Aluminiumoxidphase neben nur noch wenig gamma-Aluminiumoxid dominierte.
Die 9 eingebauten und markierten Katalysatorproben gemäß Tabelle 3 bzw. Tabelle 4, wurden zonenförmig abgesaugt und einzeln untersucht. Die Ergebnisse sind in Tabelle 5 zusammengefaßt.
Die Ergebnisse verdeutlichen, daß die mit den 4 erfindungsgemäßen Kombinationsmerkmalen ausgestatteten Trägerkatalysatoren (Katalysatorproben R, U und X gemäß Tabelle 3 bzw. Tabelle 4) eine gute Widerstandsfähigkeit gegenüber Dauereinwirkung erhöhter Temperaturen unter Oxichlorierungsbedingungen, das heißt Freisetzen von Reaktionswärme an den Katalysatorpartikeln, aufweisen. Nach achtmonatiger Temperatureinwirkung beträgt der Verlust an Bruchhärte im Durchschnitt nur 24 % bezogen auf die Ausgangswerte, wohingegen die Katalysatorproben S, T, V, W, Y, Z - wie in Tabelle 6 festgehalten - durch Nichterfüllung eines Kombinationsmerkmals Porosität bzw. $h/d_ä$-Wert gegenüber den erfindungsgemäßen Katalysatorproben unter vergleichbaren Temperatur- und Reaktionsbedingungen im gleichen Zeitraum eine im Schnitt 62 %ige Verschlechterung der Bruchhärte erleiden, wobei der Verlust an Bruchhärte durchschnittlich 39 % bezogen auf die jeweiligen Ausgangswerte beträgt. Dies ist überraschend, weil die Ergebnisse des Beispiel 1 gemäß Tabelle 2 demonstrieren, daß allein die Zugabe des erfindungsgemäßen Temperaturstabilisatorgemisches Cerdioxid/Zirkondioxid dem gamma-Aluminiumoxid-Träger eine beträchtliche Formbeständigkeit und Wärmefestigkeit verleiht.

Offensichtlich ist es jedoch unter den Bedingungen der Oxichlorierungsreaktion, bei denen punktuell an den Katalysatorpartikeln zusätzlich zur eingebrachten Wärme über die Reaktionspartner bzw. Reaktionsprodukte eine beträchtliche Reaktionswärme freigesetzt wird, für eine gute Temperaturstabilität erforderlich, daß zusätzlich auch thermodynamisch wie reaktionskinetisch vorteilhafte kurze Diffusionswege im Bereich der eigentlichen katalytischen Reaktionszone zugegen sind. Insbesondere um Verkokungen durch Ausbildung von Hot-spots möglichst zu verhindern, die wiederum die Wärmebeständigkeit trotz Vorhandenseins von Temperaturstabilisatoren insgesamt beeinträchtigen bzw. durch vermehrte Ausbildung der alpha-Aluminiumoxidphase die Bruchhärte vermindern. Somit kommt es durch die sprengende Wirkung der Kokseinlagerungen zu einem relativ raschen Zerfall der Katalysatorformlinge, obwohl letztere durch erfindungsgemäße Formgebungen den Wärmeaustauschkoeffizienten zwischen Katalysatorkörper und Produktgasstrom fördern und somit eigentlich eine optimale Wärmeabführung in den Produktstrom erfolgt.

Tabelle 5:

Wärmebeständigkeit von Trägerkatalysatoren gemäß Tabelle 3 bzw. Tabelle 4 unter den Betriebsbedingungen des ersten Reaktors einer Luft-Oxichlorierung mit Dreireaktortechnik

| Katalysator Bezeich-nung | Reaktor 1 | Bruchhärte (N) | | Bruchhärte-verlust (%) | $Al_2O_3$-Phasen | | Farbe | Visuelle Beurteilung | | |
|---|---|---|---|---|---|---|---|---|---|---|
| | | vorher | nachher | | $\gamma$-$Al_2O_3$ | $\alpha$-$Al_2O_3$ | | Bruch | Staub | Verkokung |
| R | Zone 1 | 44 | 36 | 18 | +++ | – | braun | – | – | – |
| | Zone 2 | 44 | 33 | 25 | +++ | + | braun/grau | + | + | + |
| | Zone 3 | 44 | 34 | 23 | +++ | – | braun/grau | + | + | + |
| | Zone 4 | 44 | 33 | 25 | +++ | + | braun/grau | + | + | + |
| | Zone 5 | 44 | 36 | 18 | +++ | – | braun | + | – | – |
| U | Zone 1 | 26 | 21 | 19 | +++ | – | braun | – | – | – |
| | Zone 2 | 26 | 18 | 31 | +++ | + | braun/grau | + | + | + |
| | Zone 3 | 26 | 19 | 27 | +++ | + | braun/grau | – | + | + |
| | Zone 4 | 26 | 18 | 31 | +++ | + | braun/grau | + | + | + |
| | Zone 5 | 26 | 20 | 23 | +++ | – | braun | + | – | – |
| X | Zone 1 | 40 | 32 | 20 | +++ | – | braun | – | – | – |
| | Zone 2 | 40 | 30 | 25 | +++ | + | braun/grau | + | + | + |
| | Zone 3 | 40 | 31 | 23 | +++ | – | braun/grau | – | + | + |
| | Zone 4 | 40 | 29 | 28 | +++ | + | braun/grau | + | + | + |
| | Zone 5 | 40 | 31 | 23 | +++ | – | braun | + | – | – |

+++ Hauptanteil / ++ Nebenanteil / + geringer Anteil/ - nicht nachweisbar

Tabelle 6:

Wärmebeständigkeit von Trägerkatalysatoren gemäß Tabelle 3 bzw. Tabelle 4 unter den
Betriebsbedingungen des ersten Reaktors einer Luft-Oxichlorierung mit Dreireaktortechnik

| Katalysator Bezeichnung | Reaktor 1 | Bruchhärte (N) | | Bruchhärte-verlust | $Al_2O_3$-Phasen | | Farbe | Visuelle Beurteilung | | |
|---|---|---|---|---|---|---|---|---|---|---|
| | | vorher | nachher | (%) | $\gamma$-$Al_2O_3$ | $\alpha$-$Al_2O_3$ | | Bruch | Staub | Verkokung |
| S/T | Zone 1 | 44 | 30 | 32 | +++ | + | braun/grau | ++ | + | + |
| | Zone 2 | 44 | 27 | 39 | +++ | +++ | schwarz | +++ | +++ | +++ |
| | Zone 3 | 44 | 29 | 34 | +++ | ++ | braun/schwarz | ++ | ++ | ++ |
| | Zone 4 | 44 | 28 | 36 | +++ | ++ | schwarz | +++ | +++ | +++ |
| | Zone 5 | 44 | 29 | 39 | +++ | ++ | braun/schwarz | ++ | ++ | ++ |
| V/W | Zone 1 | 26 | 16 | 38 | +++ | + | braun/schwarz | ++ | ++ | ++ |
| | Zone 2 | 26 | 13 | 50 | +++ | ++ | schwarz | +++ | +++ | +++ |
| | Zone 3 | 26 | 15 | 42 | +++ | + | schwarz | +++ | +++ | +++ |
| | Zone 4 | 26 | 14 | 46 | +++ | ++ | schwarz | +++ | +++ | +++ |
| | Zone 5 | 26 | 16 | 38 | +++ | ++ | braun/schwarz | ++ | ++ | ++ |
| Y/Z | Zone 1 | 40 | 27 | 33 | +++ | + | braun/grau | ++ | + | + |
| | Zone 2 | 40 | 24 | 40 | +++ | +++ | schwarz | +++ | +++ | +++ |
| | Zone 3 | 40 | 25 | 38 | +++ | ++ | braun/schwarz | ++ | ++ | ++ |
| | Zone 4 | 40 | 24 | 40 | +++ | ++ | schwarz | +++ | +++ | +++ |
| | Zone 5 | 40 | 26 | 35 | +++ | ++ | braun/schwarz | ++ | ++ | ++ |

+++ Hauptanteil / ++ Nebenanteil / + geringer Anteil/ - nicht nachweisbar

EP 0 920 908 A1

Beispiel 3 (Vergleichsbeispiel 1)

Analog Beispiel 2 werden die Katalysatorproben R, U und Z gemäß Tabelle 3 - jedoch jeweils ohne Zusatz des Temperaturstabilisators Cerdioxid/Zirkondioxid -, beladen mit den Aktivkomponenten gemäß Tabelle 4 mit analogem Füllplan in 3 gleichmäßig über den Querschnitt von Reaktor 1 verteilte Rohre eingefüllt, während die restlichen 3197 Rohre mit Katalysatoren gemäß EP 0 461 431 gefüllt sind. Die Reaktionsbedingungen und die Belastung der Anlage sind analog Beispiel 2. Nach nur 6 Monaten mußte wegen Anstieg des Druckabfalls der Katalysator im 1. Reaktor gewechselt werden. Die aus den 3 Proberohren abgesaugten Probekatalysatoren zeigten in allen 5 Zonen dominantes Bruchverhalten mit viel Grob- und Feinstaubanteil sowie deutliche Verkokungen vor allem im Bereich der zwei kamelbuckelartigen axialen Hot-spot-Zonen.
Der Zusatz von Yttrium-III-chlorid allein bewirkt also trotz Vorhandenseins von thermodynamisch und reaktionskinetisch vorteilhaften kurzen Diffusionswegen, die sich durch die erfindungsgemäßen Einzelmerkmale Porosität bzw. $h/d_ä$-Wert ergeben, bzw. trotz optimaler Wärmeabführung durch die beanspruchten Formgeometrien keinen temperaturstabilisierenden Effekt.

Beispiel 4

Der verwendete Laborreaktor besteht gemäß Figur 7 aus einem senkrecht stehenden Nickelrohr (1) mit 25 mm lichter Weite und 2000 mm Länge und ist von einem Stahlmantel (2) umgeben. Der Reaktor verfügt über drei Zuleitungen, wobei die Zuleitungsstelle (3) am oberen Ende des Reaktionsrohres angeordnet ist, während die Zuleitungsstellen (4) und (5) nach dem ersten bzw. zweiten Drittel des Reaktionsrohres angeordnet seitlich angebracht sind. Im Hohlraum (6) zwischen Nickelrohr (1) und Stahlrohr (2), der vertikal in drei gleich lange Segmente aufgeteilt ist, wird thermostatisiertes Siliconöl unterschiedlicher Temperatur in drei Heiz-/Kühlkreisläufen I bis III umgepumpt.
Die drei Kreisläufe I bis III sind über die Reglerelemente (7,8,9) jeweils separat temperaturregelbar.
Das Reaktionsrohr wird nach folgendem Füllplan jeweils mit den Trägerkatalysatoren R bis Z gemäß Tabelle 3 bzw. Tabelle 4 (von oben nach unten betrachtet) beschickt:

130 mm   Berlsättel (Inertmaterial) mit 4 mm Durchmesser
235 mm   Katalysator R bis Z jeweils Typ A mit 7,9 Gew.% $CuCl_2$, 3,95 Gew.-% KCl und 0,29 Gew.-% $YCl_3$
115 mm   Kataysator R bis Z jeweils Typ B mit 12,0 Gew.-% $CuCl_2$, 3,6 Gew.-% KCl und 0,44 Gew.-% $YCl_3$
115 mm   Katalysator R bis Z jeweils Typ C mit 22,5 Gew.-% $CuCl_2$, 2,1 Gew.-% KCl und 0,82 Gew.-% $YCl_3$
115 mm   Katalysator R bis Z jeweils Typ D mit 22,5 Gew.-% $CuCl_2$, 3,75 Gew.-% KCl und 0,82 Gew.-% $YCl_3$
350 mm   Katalysator R bis Z jeweils Typ B mit 12,0 Gew.-% $CuCl_2$, 3,6 Gew.-% KCl und 0,44 Gew.-% $YCl_3$
230 mm   Katalysator R bis Z jeweils Typ C mit 22,5 Gew.-% $CuC_2$, 2,1 Gew.-% KCl und 0,82 Gew.-% $YCl_3$
580 mm   Katalysator R bis Z jeweils Typ C mit 22,5 Gew.-% $CuCl_2$, 2,1 Gew.-% KCl und 0,82 Gew.-% $YCl_3$
130 mm   Bersättel (Inertmaterial) mit 4 mm Durchmesser

Die Herstellung der verwendeten Katalysatoren ist in Beispiel 2 eingehend beschrieben. Die spezifischen Oberflächen der temperaturstabilisierten Träger liegen bei Werten zwischen 180 und 250 $m^2/g$. Die Porendurchmesser bewegen sich dominant im Bereich von 4 - 20 nm. Die einzelnen Gasströme werden über geeichte Rotameter zugeführt. 50 Nl/h Chlorwasserstoff und 26,5 Nl/h Ethylen werden zunächst vermischt und dann zusammen mit 26 Nl/h Luft über die Zuleitungsstelle (3) am Reaktoroberteil aufgegeben. Weitere 26 Nl/h Luft bzw. 13 Nl/h Luft werden über die Zuleitungen (4) bzw (5) zugegeben. Das den Reaktor (1) über Leitung (10) verlassende gasförmige Reaktionsgemisch wird im Kühler 11 mit Wasser gekühlt, wobei Partialkondensation eintritt. Im Abscheider (12) wird die flüssige Phase, bestehend aus rohem 1,2-Dichlorethan (EDC) und Wasser, in dem nicht umgesetzter Chlorwasserstoff größtenteils gelöst ist, abgetrennt. Der nicht kondensierbare Gasstrom wird in der Kältefalle (13) auf - 25°C abgekühlt, wobei weitere Kondensation eintritt und dann im anschließenden Wasserwäscher (14) Chlorwasserstofffrei gewaschen.
Die beiden Kondensate aus dem Abscheider (12) und der Kältefalle (13) werden vereint und nach Abtrennen der wässrigen Phase durch Dekantieren gaschromatographisch analysiert. Das Abgas nach der Kältefalle (13) wird nach Probenahme über die Gasmaus (15) gaschromatographisch auf CO und $CO_2$ untersucht.
Der Chlorwasserstoff-Umsatz wird aus dem titrimetrisch ermittelten Chlorwasserstoffgehalt in der vereinten wässrigen Phase und im Ablauf des Wasserwäschers (14) errechnet. Die Reaktion wird bei Atmosphärendruck betrieben. Die Ergebnisse dieser Versuch sind in Tabelle 7 aufgelistet.
Demzufolge sind die mit den beanspruchten 4 kombinativen Erfindungsmerkmalen ausgestatteten Trägerkatalysatoren R,U und X am aktivsten und selektivsten, erkennbar durch erhöhte HCl-Umsatzraten bei relativ niedrigen Reaktortemperaturen und geringen Überschüssen an Ethylen und Luft, jeweils bezogen auf die Stöchiometrie zu Chlorwasserstoff bzw. durch niedrige Nebenproduktbildung und Ethyltotaloxidationsrate bei relativ niedrigen Hot-spot-Temperaturen im 1. Reaktor.

Wie die Ergebnisse der Trägerkatalysatoren S, V und Y demonstrieren, wird schon allein bei Nichterfüllung des erfindungsgemäßen Einzelmerkmals Porosität die Aktivität und Selektivität deutlich schlechter, was überrascht, weil üblicherweise mit niedrigerem Mesoporenanteil bzw. entsprechend reduziertem Porenvolumen in Verbindung mit den beanspruchten Katalysatordimensionierung normalerweise die katalytische Wirksamkeit verbessert wird. Die Ergebnisse der Trägerkatalysatoren T, W und Z, bei denen nur das erfindungsgemäße Einzelkriterium $h/d_{\ddot{a}}$ nicht erfüllt ist, zeigen bei guter Aktivität einen deutlichen Abfall an der Selektivität vor allem bzgl. Ethylentotaloxidationsrate.

Daß die aktivitäts- und selektivitätssteigernde Wirkung von Yttrium-III-chlorid nur in Kombination mit den beanspruchten Erfindungsmerkmalen Porosität und $h/_{\ddot{a}}$-Wert voll zum Ausdruck kommt, konnte von keinem Fachmann vorausgesagt werden, zumal die Wirkung von Yttrium-III-chlorid per se wegen der Ausbildung gasundurchlässiger Oxidfilme schon in der Literatur angezweifelt worden ist.

Beispiel 5 (Vergleichsbeispiel 2)

Es wird die in Beispiel 4 beschriebene Laborversuchsapparatur benutzt. Folgende Katalysatoren kommen zum Einsatz, wobei jeweils der in Beispiel 4 zitierte Füllplan sinngemäß verwendet wird.

Katalysator 1

| | |
|---|---|
| Trägermaterial: | $\gamma$-$Al_2O_3$ mit 5 Gew.-% $CeO_2$ und 0,5 Gew.-% $ZrO_2$, Porenvolumen 0,8 $cm^3$/g, $h/d_{\ddot{a}}$ = 0,5, Wagenradform |
| Typ A: | mit 7,9 Gew.-% $CuCl_2$, 3,95 Gew.-% KCl und 0,36 Gew.-% $LaCl_3$ |
| Typ B: | mit 12,0 Gew.-% $CuCl_2$, 3,6 Gew.-% KCl und 0,55 Gew.-% $LaCl_3$ |
| Typ C: | mit 22,5 Gew.-% $CuCl_2$, 2,1 Gew.-% KCl und 1,03 Gew.-% $LaCl_3$ |
| Typ D: | mit 22,5 Gew.-% $CuCl_2$, 3,75 Gew.-% KCl und 1,03 Gew.-% $LaCl_3$ |

Katalysator 2

| | |
|---|---|
| Trägermaterial: | $\gamma$-$Al_2O_3$ mit Porenvolumen 0,8 $cm^3$/g, $h/d_{\ddot{a}}$ = 0,5, Wagenradform |
| Typ A: | mit 7,9 Gew.-% $CuCl_2$, 3,95 Gew.-% Kcl und 0,29 Gew.-% $YCl_3$ |
| Typ B: | mit 12,0 Gew.-% $CuCl_2$, 3,6 Gew.-% KCl und 0,44 Gew.-% $YCl_3$ |
| Typ C: | mit 22,5 Gew.-% $CuCl_2$, 2,1 Gew.-% KCl und 0,82 Gew.-% $YCl_3$ |
| Typ D: | mit 22,5 Gew.-% $CuCl_2$, 3,75 Gew.-% Kcl und 0,82 Gew.-% $YCl_3$ |

Katalysator 3

| | |
|---|---|
| Trägermaterial: | $\gamma$-$Al_2O_3$, Porenvolumen 0,8 $cm^3$/g, $h/d_{\ddot{a}}$ = 0,5, Wagenradform |
| Typ A: | mit 7,9 Gew.-% $CuCl_2$, 3,95 Gew.-% KCl |
| Typ B: | mit 12,0 Gew.-% $CuCl_2$, 3,6 Gew.-% KCl |
| Typ C: | mit 22,5 Gew.-% $CuCl_2$, 2,1 Gew.-% KCl |
| Typ D: | mit 22,5 Gew.-% $CuCl_2$, 3,75 Gew.-% KCl |

Die Belastung an Chlorwasserstoffgas beträgt jeweils 50 Nl/h. Die Zugabe von Ethylen und Gesamtluft errechnet sich aus den in Tabelle 8 jeweils angegebenen stöchiometrischen Überschußzahlen, wobei die Aufteilung der Gesamtluft auf die 3 Reaktoren analog Beispiel 4 40/40/20 % beträgt. In Tabelle 8 sind neben Angaben über die jeweiligen angewandten Reaktionstemperaturen bzw. Hot-spot-Temperaturen im 1. Reaktor auch die Versuchsergebnisse zusammengefaßt.

Die Ergebnisse verdeutlichen, daß Lanthan-III-chlorid im Vergleich zum Yttrium-III-chlorid (Tabelle 7) bei vergleichbaren Reaktionstemperaturen und Ethylen- bzw. Luftüberschüssen etwas weniger aktiv, aber wesentlich weniger selektiv wirkt, vorallem hinsichtlich der Ethylentotaloxidationsrate, die durch die Ausbildung einer besonders hohen Hot-spot-Temperatur in Reaktor 1 enorm ansteigt. Dies beruht darauf, daß Lanthan-III-chlorid im Gegensatz zum Yttrium-III-chlorid kein richtiger Aktivator ist, der die Reaktionsrate durch Herabsetzen der Aktivierungsenergie katalysiert (entspricht einer Absenkung der Temperatur im Bereich der eigentlichen Reaktionszone des Katalysators), sondern nur den preexpontiellen Faktor erhöht, wodurch sich zwar die Stoßzahl der zur Reaktion kommenden Moleküle vermehrt (entspricht einer gewissen Aktivitätssteigerung), dafür aber an den Katalysatorpartikeln heisse Temperaturnester (Hotspots) auftreten, die die Selektivität stark schmälern.

Die Ergebnisse der Katalysatoren 2 bzw. 3 verdeutlichen, daß die erfindungsgemäßen Trägerkatalysatoren ohne den Zusatz von Yttrium-III-chlorid weniger aktiv und selektiv sind.

Um nämlich einen HCl-Umsatz von etwa 99,5 % zu erzielen ist bei Abwesenheit des Promotors Yttrium-III-chlorid eine Erhöhung der Reaktinstemperaturen und der Ethylen- bzw. Luftüberschüsse erforderlich. Beide Maßnahmen führen zwangsweise zu einer Selektivitätsverschlechterung, da erhöhte Temperaturen generell die Substitutionsreaktionen bzw. Spaltreaktionen (1,1,2-Trichlorethan-Bildung, $C_1$-CKW-Bildung durch C-C-Kettenspaltung) begünstigen bzw. erhöhte Ethylen- und Sauerstoffpartialdrücke im Reaktionsgemisch besonders bei Temperaturerhöhung die Ethylentotaloxidationsrate fördern.

Tabelle 7:

Ergebnisse des Beispiels 4

| Kata-ly-sator | Kreislauf-temperaturen | | | Ethylen-Excess % | Luft* Excess % | HCl-Umsatz % | Selektivität zu | | | | | CO* mol% | Hot Spot im 1. Reaktor °C |
| | I °C | II °C | III °C | | | | EDC mol% | Chloral mol% | Chloro-form mol % | Tetra-chlor-Kohlen-stoff mol% | 1,1,2-Trichlo rethan mol% | | |
|---|---|---|---|---|---|---|---|---|---|---|---|---|---|
| R | 190 | 200 | 210 | 6 | 9 | 99,5 | 97,4 | 0,02 | 0,03 | 0,03 | 0,2 | 2,2 | 270 |
| S | 190 | 200 | 210 | 6 | 9 | 99,0 | 96,6 | 0,02 | 0,04 | 0,04 | 0,5 | 2,7 | 298 |
| T | 190 | 200 | 210 | 6 | 9 | 99,4 | 96,5 | 0,02 | 0,04 | 0,04 | 0,5 | 2,8 | 300 |
| U | 190 | 200 | 210 | 6 | 9 | 99,3 | 97,2 | 0,02 | 0,03 | 0,03 | 0,3 | 2,3 | 275 |
| V | 190 | 200 | 210 | 6 | 9 | 89,8 | 96,4 | 0,02 | 0,05 | 0,05 | 0,5 | 2,9 | 300 |
| W | 190 | 200 | 210 | 6 | 9 | 99,2 | 96,3 | 0,02 | 0,04 | 0,04 | 0,5 | 3,0 | 302 |
| X | 190 | 200 | 210 | 6 | 9 | 99,4 | 97,3 | 0,02 | 0,03 | 0,03 | 0,2 | 2,3 | 273 |
| Y | 190 | 200 | 210 | 6 | 9 | 89,9 | 96,5 | 0,02 | 0,04 | 0,04 | 0,5 | 2,8 | 299 |
| Z | 190 | 200 | 210 | 6 | 9 | 99,3 | 96,4 | 0,02 | 0,04 | 0,04 | 0,5 | 2,9 | 301 |

*jeweils bezogen auf die Stöchiometrie zu Chlorwasserstoff

Tabelle 8:

Ergebnisse des Beispiels 5

| Katalysator | Kreislauftemperaturen | | | Ethylen-Excess % | Luft* Excess % | HCl-Umsatz % | EDC mol% | Selektivität zu | | | | | Hot Spot im 1.Reaktor °C |
| | I °C | II °C | III °C | | | | | Chloral mol% | Chloroform mol % | Tetrachlor-Kohlenstoff mol% | 1,1,2-Trichlorethan mol% | CO mol% | |
|---|---|---|---|---|---|---|---|---|---|---|---|---|---|
| 1 | 190 | 200 | 210 | 6 | 9 | 99,0 | 95,0 | 0,08 | 0,04 | 0,04 | 0,4 | 4,3 | 320 |
| 2 | 190 | 200 | 210 | 6 | 9 | 99,5 | 97,4 | 0,02 | 0,03 | 0,03 | 0,2 | 2,2 | 270 |
| 3 | 200 | 210 | 220 | 12 | 18 | 99,0 | 92,0 | 0,16 | 0,08 | 0,10 | 1,1 | 6,4 | 307 |

*jeweils bezogen auf die stöchiometrie zu Chlorwasserstoff

Vergleicht man die Ergebnisse von Tabelle 7 und der Katalysatoren 2 und 3 in Tabelle 8 so kann man feststellen, daß Yttrium-III-chlorid speziell die Choralbildung stark unterdrückt, wodurch für dessen notwendige kaustische Spaltung zu Chloroform und Natriumformiat bei der alkalischen Wäsche des erzeugten Roh-EDC's weit weniger Lauge verbraucht wird.

Beispiel 6

Es wird das in Beispiel 4 beschriebene und in Figur 7 gezeigte Dreireaktorensystem verwendet. Zum Einsatz kommt der erfindungsgemäße Trägerkatalysator R gemäß Tabelle 3 und 4 mit Füllplan analog Beispiel 4. Die Reaktionsbedingungen sind ebenfalls analog Beispiel 4 mit der Ausnahme, daß anstelle von Luft reiner Sauerstoff verwendet wird. Um die technische Kreisgasfahrweise im ethylenreichen Bereich gemäß US-PS 3,892,816 wegen Fehlens eines Kreisgasverdichters annähernd zu simulieren, werden folgende Mengen an Reaktanden eingesetzt:

50 Nl/h Chlorwasserstoff, vermischt mit 95 Nl/h Ethylen und
5,3 Nl/h Sauerstoff über Zuleitstelle (3)
5,3 Nl/h Sauerstoff über Zuleitstelle (4)
2,3 Nl/h Sauerstoff über Zuleitstelle (5)

Diese Feed-Einstellung entspricht einem Sauerstoffüberschuß von 3,2 % bzw. einem Ethylenüberschuß von 380 %, jeweils bezogen auf die Stöchiometrie zu Chlorwasserstoff.
Die Aufarbeitung und analytische Auswertung der Reaktionsprodukte erfolgt gemäß Beispiel 4.
Folgende Ergebnisse wurden bei Temperaturen von

Kreislauf I      bei 190°C
Kreislauf II     bei 200°C
Kreislauf II     bei 200°C

erzielt:

Hot-spot-Temperatur in R-301:
252°C
HCl-Umsatz:
99,8 %

Selektivität zu

98,6 mol% EDC
0,01 mol% Chloral
0,02 mol% Chloroform
0,02 mol% Tetrachlorkohlenstoff
0,05 mol% 1,1,2-Trichlorethan
1,2 mol% $CO_x$

Vergleicht man die Meßergebnisse mit denen von Beispiel 4 (Tabelle 7) so kommt die überlegene Technologie der Sauerstoffkreisgasfahrweise mit hohem Ethylen-Gehalt im Kreisgas, vorallem hinsichtlich Aktivität und Selektivität deutlich zum Ausdruck. Darüberhinaus wird aufgrund der niedrigen Hot-spot-Temperaturen durch die Sauerstoffkreisgasfahrweise auch die Wärmefestigkeit bzw. Langzeitstabilität der erfindungsgemäß beanspruchten Trägerkatalysatoren noch wesentlich verbessert.

**Patentansprüche**

1.  Trägerkatalysator aufweisend

a) 0,5 - 15 Gew.-% einer oder mehrerer $Cu^{II}$-Verbindungen, wobei sich die Mengenangabe auf Kupfermetall bezieht,
b) 0,1 - 8 Gew.-% einer oder mehrerer Alkalimetallverbindungen, wobei sich die Mengenangabe auf Alkalimetall bezieht,
c) 0,1 - 10 Gew.-% eines Oxidgemisches, welches aus
c1) 80 - 95 Mol % von Oxiden der Ceriterden mit den Ordungszahlen 57 bis 62, ausgenommen Promethium, und

c2) 5 - 20 Mol % Zirkondioxid, besteht, wobei c1) und c2) zusammen 100 Mol % ergeben müssen und wobei sich die Mengenangabe von c) auf die Oxide des Gemisches bezieht, und

d) den Rest auf 100 Gew.-% als Trägermaterial $\gamma$- und/oder $\vartheta$-Aluminiumoxid;

wobei

e) das Trägermaterial d) ein Gesamtporenvolumen im Bereich von 0,65 bis 1,2 cm$^3$/g aufweist; und wobei

f) der Trägerkatalysator als zylindrischer Hohlkörper mit wenigstens einem Durchtrittskanal vorliegt, wobei für Durchmesser $d_a$ von bis zu 6 mm das Verhältnis von Höhe h zu Außendurchmesser $d_a$ kleiner 1,5 ist und für Durchmesser $d_a$ über 6 mm das Verhältnis h/$d_a$ kleiner 0,6 ist.

2. Trägerkatalysator nach Anspruch 1,
   **dadurch gekennzeichnet**, daß das molare Verhältnis von Cu$^{II}$-Verbindungen a) zu Alkalimetall-Verbindungen b) im Bereich von 1 : 1 bis 8 : 1 ist.

3. Trägerkatalysator nach Anspruch 1 oder 2,
   **dadurch gekennzeichnet**, daß er 2 - 8 Gew.% Oxidgemisch c) aufweist.

4. Trägerkatalysator nach einem oder mehreren der vorgenannten Ansprüche,
   **dadurch gekennzeichnet**, daß das Oxidgemisch c) aus 80 - 90 Mol % c1) und 10 - 20 Mol % c2) besteht.

5. Trägerkatalysator nach einem oder mehreren der vorgenannten Ansprüche,
   **dadurch gekennzeichnet**, daß die Cu$^{II}$-Verbindung CuCl$_2$ ist.

6. Trägerkatalysator nach einem oder mehreren der vorgenannten Ansprüche,
   **dadurch gekennzeichnet**, daß die Alkalimetallverbindung KCl ist.

7. Trägerkatalysator nach einem oder mehreren der vorgenannten Ansprüche,
   **dadurch gekennzeichnet**, daß c1) CeO$_2$ ist.

8. Trägerkatalysator nach einem oder mehreren der vorgenannten Ansprüche,
   **dadurch gekennzeichnet**, daß d) $\gamma$-Al$_2$O$_3$ ist.

9. Trägerkatalysator nach einem oder mehreren der vorgenannten Ansprüche,
   **dadurch gekennzeichnet**, daß 80 % des Gesamtporenvolumens Mesoporen mit einem Durchmesser im Bereich von 4 bis 20 nm sind.

10. Trägerkatalysator nach einem oder mehreren der vorgenannten Ansprüche,
    **dadurch gekennzeichnet**, daß die zylindrischen Hohlkörper, Hohlextrudate, Wagenräder mit 2 bis 12 Speichen und/oder Monolithformkörper sind.

11. Trägerkatalysator nach Anspruch 10,
    **dadurch gekennzeichnet**, daß die Außendurchmesser im Bereich von 4 bis 10 mm sind.

12. Trägerkatalysator nach einem oder mehreren der vorgenannten Ansprüche,
    **dadurch gekennzeichnet**, daß die zylindrischen Hohlkörper Wagenräder oder Monolithformkörper sind und daß h/$d_a$ ≤ 0,6 ist.

13. Trägerkatalysator nach einem oder mehreren der vorgenannten Ansprüche,
    **dadurch gekennzeichnet**, daß er zusätzlich einen Gehalt einer Yttrium-III-Verbindung aufweist.

14. Trägerkatalysator nach Anspruch 13,
    **dadurch gekennzeichnet**, daß er 0,5 bis 10 Mol % Yttrium-III-Verbindung bezogen auf den molaren Gehalt an Kupfer-II-Verbindung(en), aufweist.

15. Trägerkatalysator nach Anspruch 14,
    dadurch gekennzeichnet, daß die Yttrium-III-Verbindung YCl$_3$ ist.

16. Verfahren zur Herstellung eines Trägerkatalysators gemäß den Ansprüchen 1 bis 15,

**dadurch gekennzeichnet**, daß man

i) Trägermaterialien d) der Geometrie f) mit löslichen Precursoren der Komponenten c1) und c2) beaufschlagt;
ii) die Precursor-Verbindungen in die oxidische Form überführt; und
iii)die oxidisch beaufschlagten Trägermaterialien mit den Komponenten a) und b) belädt.

17. Verfahren zur Herstellung eines Trägerkatalysators gemäß den Ansprüchen 1 bis 15,
**dadurch gekennzeichnet**, daß man

iv) eine Mischung aus den Komponenten c) und d) zur Geometrie f) formt; und
v) die geformten Trägermaterialien mit den Komponenten a) und b) belädt.

18. Verwendung der Trägerkatalysatoren gemäß den Ansprüchen 1 bis 15 zur Oxichlorierung von Ethylen.

19. Verfahren zur Herstellung von 1,2-Dichlorethan durch Oxichlorierung von Ethylen mit Chlorwasserstoff unter Verwendung des Trägerkatalysators gemäß den Ansprüchen 1 bis 15, wobei Luft oder mit Sauerstoff angereicherte Luft, oder reiner Sauerstoff unter Kreisgasführung des überschüssigen nicht umgesetzten Ethylens bei hochethylenhaltigem Kreisgas, als Oxidationsmittel dient, und das Verfahren einstufig oder mehrstufig erfolgt, wobei die Temperatur zwischen 220°C und 320°C bei einem Druck von 3 bis 10 bar absolut beträgt.

**Figur 1**  Querschnitt ringförmiges Hohlextrudat

**Figur 3**  Querschnitt Minilith

$d_2$  $d_1$

$d_2$  $d_1$  $d_3$

**Figur 2**   Querschnitt Wagenradform

**Figur 5**   Wagenradform perspektivisch

**Figur 4**   Ringförmiges Hohlextrudat perspektivisch

**Figur 6**   Minilithform perspektivisch

30

Figur 7

Europäisches
Patentamt

**Nummer der Anmeldung**

EP 98 12 1259

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,A | DE 17 68 453 A (SHELL INTERNATIONALE RESEARCH MAATSCHAPPIJ N.V.) 18. März 1971 <br><br> * Ansprüche 1-3 * <br> * Seite 2, Zeile 1 - Seite 3, Zeile 11 * <br> * Beispiel 1 * <br> --- | 1-6, 13-15, 18,19 | B01J23/83 <br> B01J35/02 <br> C07C17/156 <br> B01J21/06 |
| D,A | J.A. ALLEN AND A.J. CLARK: "Oxychlorination catalysts" REVIEWS OF PURE AND APPLIED CHEMISTRY, Bd. 21, September 1971, Seiten 145-166, XP002094396 <br> * Tabelle 1 * <br> * Seite 150, linke Spalte, Zeile 9 - rechte Spalte, Zeile 25 * <br> * Seite 152, rechte Spalte, Zeile 4 - Seite 154, rechte Spalte, Zeile 3 * <br> --- | 1,2,5-9, 13-15, 18,19 | |
| D,A | WO 96 40431 A (DOW CHEMICAL CO) 19. Dezember 1996 <br><br> * Ansprüche 1,3,8,9 * <br> * Seite 2, Zeile 37 - Seite 6, Zeile 10 * <br> * Abbildung 1 * <br> --- | 1,2,5,6, 8-12,16, 18,19 | **RECHERCHIERTE SACHGEBIETE (Int.Cl.6)** <br><br> B01J <br> C07C |
| D,A | US 4 446 249 A (EDEN JAMAL S) 1. Mai 1984 <br><br> * Ansprüche 1,2,7,8,10 * <br> * Beispiele 1,3,5,6 * <br> --- | 1,2,5,6, 8,16,18, 19 | |
| D,A | EP 0 582 165 A (GEON CO) 9. Februar 1994 <br><br> * Ansprüche 1,3,4,10-15 * <br> * Beispiele V,VII * <br> --- <br><br> -/-- | 1,2,5-8, 18,19 | |

Der vorliegende Recherchenbericht wurde für alle Patentansprüche erstellt

| Recherchenort | Abschlußdatum der Recherche | Prüfer |
|---|---|---|
| DEN HAAG | 24. Februar 1999 | Zuurdeeg, B |

Europäisches
Patentamt

**EUROPÄISCHER RECHERCHENBERICHT**

Nummer der Anmeldung

EP 98 12 1259

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | DATABASE WPI<br>Section Ch, Week 9540<br>Derwent Publications Ltd., London, GB;<br>Class E19, AN 95-309022<br>XP002093878<br>& JP 07 206726 A (ASAHI GLASS CO LTD)<br>, 8. August 1995<br>* Zusammenfassung *<br>---- | 1,2,5-7,<br>18 | |
| A | US 3 914 390 A (KUDO TETSUICHI ET AL)<br>21. Oktober 1975<br>* Ansprüche 1,2,5,6 *<br>* Spalte 2, Zeile 45 – Spalte 3, Zeile 2 *<br>* Spalte 4, Zeile 62 – Spalte 6, Zeile 14<br>*<br>* Beispiel 5 *<br>----- | 1,8,10,<br>11,16 | |

RECHERCHIERTE
SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| DEN HAAG | 24. Februar 1999 | Zuurdeeg, B |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**                    EP 98 12 1259

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-02-1999

| Im Recherchenbericht angeführtes Patentdokument | Datum der Veröffentlichung | Mitglied(er) der Patentfamilie | Datum der Veröffentlichung |
|---|---|---|---|
| DE 1768453 A | 18-03-1971 | NL 6706807 A<br>BE 715159 A<br>DE 1793547 A<br>FR 1580937 A<br>GB 1167280 A | 25-07-1967<br>18-11-1968<br>02-03-1972<br>12-09-1969<br>15-10-1969 |
| WO 9640431 A | 19-12-1996 | AU 5311896 A<br>CA 2218744 A<br>EP 0830204 A<br>NO 975699 A | 30-12-1996<br>19-12-1996<br>25-03-1998<br>05-12-1997 |
| US 4446249 A | 01-05-1984 | AR 223552 A<br>AU 539334 B<br>AU 6576980 A<br>BE 885929 A<br>BR 8008903 A<br>CA 1151636 A<br>EP 0039723 A<br>IN 151951 A<br>JP 3039740 B<br>JP 56501440 T<br>PT 72014 B<br>WO 8101284 A | 31-08-1981<br>20-09-1984<br>22-05-1981<br>16-02-1981<br>25-08-1981<br>09-08-1983<br>18-11-1981<br>10-09-1983<br>14-06-1991<br>08-10-1981<br>24-09-1981<br>14-05-1981 |
| EP 0582165 A | 09-02-1994 | US 5292703 A<br>AU 4196293 A<br>BR 9303008 A<br>CA 2101388 A<br>CN 1084430 A<br>CN 1141282 A<br>DE 69310831 D<br>DE 69310831 T<br>ES 2104004 T<br>GR 3023509 T<br>HU 67538 A,B<br>JP 6170230 A<br>MX 9304529 A<br>NO 932706 A<br>US 5382726 A | 08-03-1994<br>03-02-1994<br>16-02-1994<br>29-01-1994<br>30-03-1994<br>29-01-1997<br>26-06-1997<br>02-01-1998<br>01-10-1997<br>29-08-1997<br>28-04-1995<br>21-06-1994<br>29-04-1994<br>31-01-1994<br>17-01-1995 |
| US 3914390 A | 21-10-1975 | JP 49052167 A<br>DE 2347154 A<br>FR 2200049 A<br>GB 1439783 A | 21-05-1974<br>11-04-1974<br>19-04-1974<br>16-06-1976 |

EPO FORM P0461

Für nähere Einzelheiten zu diesem Anhang : siehe Amtsblatt des Europäischen Patentamts, Nr.12/82